# EUROPEAN PATENT APPLICATION

(11) **EP 4 744 581 A1**
(43) Date of publication of application: **20.05.2026**
(21) Application number: 25215359.8
(22) Date of filing: 12.11.2025
(51) Int. Cl.: A61B 5/01, A61B 5/0205, A61B 5/024, A61B 5/0531, A61B 5/0533, A61B 5/107, A61B 5/00

(54) **SYSTEM FOR DETECTING INFLAMMATION OF AN ANATOMICAL PART, THERAPEUTICAL SYSTEM COMPRISING SAID DETECTING SYSTEM, THERAPEUTICAL SYSTEM AND PROCESS FOR DETECTING AN INFLAMMATION STATE**

(30) Priority: 13.11.2024 IT 202400025584
(71) Applicant: Mainardi, Giuseppina, 30171 Mestre (VE) (IT)
(72) Inventor: Mainardi, Giuseppina, 30171 Mestre (VE) (IT)
(74) Representative: Bonatto, Marco

(57) **Abstract**

The system (1) according to the invention for detecting inflammation in an anatomical part of a human or animal body, comprises a plurality of sensors of at least two types, wherein said types are selected from the following list: a body temperature sensor (300), a muscular contracture and/or electromyographic sensor (302), a tumefaction and/or swelling and/or anomalous liquid infiltration sensor, a heart rate sensor (500), a blood haemoglobin oxygen saturation sensor (502), a skin perspiration sensor, a skin electrical conductance or electrodermal activity sensor, a sweating sensor, a sensor for detecting skin redness or, more generally, colour. The system (1) allows to store, share, and compare data from multiple patients to measure therapies, conduct clinical studies, refine the inflammation diagnoses themselves, and/or activate therapies even preventively by artificial intelligence tools.

## Description

### Field of the invention

The present invention relates to a system for detecting inflammation of an anatomical part, such as, for example, a wrist, an ankle, an arm, a leg, a shoulder, a hip, the sacroiliac joints, or the neck.

Such system is particularly adapted to recognize and/or monitor over time ongoing, latent, or imminent inflammation, and to track the evolution of an inflammation over time, thereby allowing earlier or improved therapeutic intervention.

The invention also relates to a process for calibrating such detection system, for example single patient-specific bio-parameters

### State of the art

In recent years, medical and biological sciences have increasingly highlighted the importance of inflammation states in many diseases.

An inflammation state can be caused, for example, by trauma or strain, chronic diseases, surgical interventions, incorrect postures, mechanical or contact-related or internally-caused actions such as contractures, fractures, abscesses, or chronic illnesses.

The Applicant, however, considers the importance of identifying and/or monitoring over time an inflammation state from its onset and even before it becomes clearly recognizable through pain or other symptoms, so as to anticipate targeted actions, preventing the inconvenience of the pain itself and possibly preventively acting before the inflammation becomes chronic: indeed, chronic inflammations are generally more painful and more difficult to treat than acute and/or irreversible ones.

An object of the present invention is to overcome the above-mentioned drawbacks and, in particular, to provide a system and a process for identifying and optionally measuring the level of inflammation in various parts of the body.

### Summary of the invention

Such object is achieved, in a first aspect of the invention, by a system for detecting inflammation of an anatomical part having the features according to claim 1.

In a particular embodiment of such detection system, said types of sensors comprise a body temperature sensor **(300)** and a muscular contracture and/or electromyographic sensor **(302).**

In a particular embodiment of such detection system, said types of sensors comprise two or more of the following types: a body temperature sensor **(300),** a heart rate sensor **(500),** a blood haemoglobin oxygen saturation sensor **(502),** a skin perspiration sensor, and a skin electrical conductance or electrodermal activity (EDA) sensor.

In a particular embodiment, such detection system, having the features of at least claim **4** or **5,** is adapted to calibrate the detections of the sensors fixed to the measurement anatomical interface **(3, 3')** or by the detection unit **(2, 2')** by taking as an index of a substantial absence of inflammation the detections of one or more body parameters acquired by one or more sensors fixed to the calibration anatomical interface **(5)** or acquired by the calibration unit **(4).**

In a particular embodiment, such detection system, having the features of at least claim **7,** is adapted to calibrate the detections of the sensors fixed to the measurement anatomical interface **(3, 3')** by subtracting the value **Var_Par(**i**)_Tar(**k), detected by at least one sensor of the calibration anatomical interface **5** from the value **Var_Par(**i**)_Ril(**k) of the at least one i-th body parameter of the same type detected at the same k-th instant by at least one of the sensors of the measurement anatomical interface **(3, 3').**

In a particular embodiment, such detection system, having the features of at least claim **7,** is adapted to calibrate the detections of a plurality of sensors of the detection unit **(2, 2')** by subtracting:
**a)** the detections **Var_Par(1)_Tar(**h), **Var_Par(2)_Tar(**h), ... **Var_Par(**i)**_Tar(**h), ... **Var_Par(**N)_**Tar(**h) of a number N of sensors and/or relating to body parameters **1, 2,** ... i... N-th, acquired at the h-th instant by the calibration unit **4** or otherwise by the sensors fixed to the calibration anatomical interface **5** on a second anatomical part; from
**b)** the detections **Var_Par(1**)**_Ril(**k), **Var_Par(2**)**_Ril(**k), ... **Var_Par(**i**)_Ril_(**k), ... **Var_Par(**N)**_Ril(**k) of a corresponding number N of sensors and/or relating to **1, 2,** ... i... N corresponding body parameters, detections acquired at the k-th instant by the detection unit **2, 2'** or otherwise by the sensors fixed to the measurement anatomical interface **3, 3'** on a first anatomical part, wherein i, h, k and N are integers equal to or greater than **1,** k is equal to or greater than h, and a detection **Var_Par(**i)**_Tar(**h) relating to an i-th body parameter of the same type relating to the corresponding detection **Var_Par(**i**)_Ril_(**k)**,** regardless of the value of h and k.

In a particular embodiment, such detection system is programmed or otherwise adapted to determine the level of inflammation of an anatomical part by implementing the following process:
**S1.1)** determining, for example for the individual subject, an admissibility range for one or more body parameters, such as for example tumefaction, liquid circulation or spill, swelling, sweating, skin coloration, skin electrical conductance or electrodermal activity, muscular contracture or temperature;
**S1.2)** signalling an anomalous intensity or magnitude of said one or more body parameters of the anatomical part of interest when the system **(1)** detects values of said one or more body parameters outside the respective admissibility range.

In a particular embodiment, such detection system, having the features of at least claim 7, is programmed or otherwise adapted to implement one or more of the following sub-operations **S8.1)-S8.7)** within operation **S1.2**)**:**
**S8.1)** determining that the temperature value measured at or in proximity to the anatomical part of interest was detected outside its normal range if it exceeds the upper admissibility threshold by at least **0.5**°C;
**S8.2)** determining that the temperature value measured at or in proximity to the anatomical part of interest was detected outside its normal range if it exceeds the upper admissibility threshold by at least **0.8-1.0**°C, for example if the anatomical part of interest is the neck, the shoulder, or one or more vertebrae;
**S8.3**) determining that the value of tumefaction, liquid circulation or spill, swelling and/or sweating at or in proximity to the anatomical part of interest was detected outside its normal range if it is at least **3.0** microsiemens below the lower admissibility threshold and/or at least **3.0** microsiemens above the upper admissibility threshold;
**S8.4**) determining that the skin colour at or in proximity to the anatomical part of interest was detected outside its normal range if the wavelength of light corresponding to the skin colour is at least **50** nanometres below the lower admissibility threshold and/or at least **50** nanometres above the upper admissibility threshold;
**S8.5**) determining that the skin colour at or in proximity to the anatomical part of interest was detected outside its normal range if its point in the LAB or CIELAB colour space is located at a distance equal to or greater than a predetermined threshold from the boundaries of the admissibility area in such colour space;
**S8.6**) determining that the blood haemoglobin oxygen saturation (SpO**2**) at or in proximity to the anatomical part of interest was detected outside its normal range if the saturation percentage is at least **2.0**% below the lower admissibility threshold;
**S8.7**) determining that muscular contracture at or in proximity to the anatomical part of interest was detected outside its normal range if it exceeds the upper admissibility threshold by at least **0.05** millivolts.

In a particular embodiment of such detection system, having the features of at least claim **4,** the calibration anatomical interface comprises one or more of a body temperature sensor **(300),** a heart rate sensor **(500),** a blood haemoglobin oxygen saturation sensor **(502),** a skin perspiration sensor, a skin electrical conductance or electrodermal activity sensor.

In a particular embodiment, such detection system, having the features of at least claim **2** and/or **3,** comprises at least two sensors of the same type for each of said at least two types, the system **(1)** being programmed or otherwise adapted to determine a gradient, a trend, or other variation as a function of the space of the body parameter determined by said at least two sensors of the same type.

In a particular embodiment, such detection system, having the features of at least claim **11,** is programmed or adapted to determine a gradient, a trend, or another variation along at least two space directions (X, Y) of the body parameter determined by said at least two sensors of the same type, wherein said at least two space directions (X, Y) are substantially orthogonal or transverse to each other.

In a second aspect of the invention, such object is achieved by a therapeutic system having the features according to claim **13.**

In a third aspect of the invention, such object is achieved by a process for detecting an inflammation state having the features according to claim **14.**

In a particular embodiment of the invention, in operation **S15.4**) of the process according to claim **14,** the detections of the sensors fixed to the measurement anatomical interface (**3, 3'**) are calibrated by taking as an index of a substantial absence of inflammation the detections of one or more body parameters acquired by one or more sensors fixed to the calibration anatomical interface (**5**).

Further features of the invention are the subject of the dependent claims.

In a fourth aspect, a further object of the invention is a therapeutic system comprising a therapeutical device **(1000),** comprising in turn at least one electrical generator **(3, 3')** configured for generating a variable electromagnetic field with a frequency between **2** and **120** Hertz and a maximum or peak, or effective, magnetic induction between **5** and **160** Gauss.

The advantages achievable with the present invention will become more apparent to a person skilled in the art from the following detailed description of some non-limiting exemplary embodiments, illustrated with reference to the following schematic figures.

### List of Figures

Figure 1 shows a functional scheme of an inflammation detection system according to a first particular embodiment of the present invention;
Figure **2** shows a front view of a first possible variant of the detection unit of the system of Figure **1****;**
Figure **3** shows a perspective view of the calibration unit of the system of Figure **1****;**
Figure **4** shows a front view of a patient's foot wearing a therapeutical device according to a first embodiment of the present invention;
Figure **5** shows a side view of the foot and the therapeutical device of Figure **4****;**
Figure **6** shows a front view of a therapeutical device according to a second embodiment of the present invention, whose anatomical support has the shape of a pair of long pants;

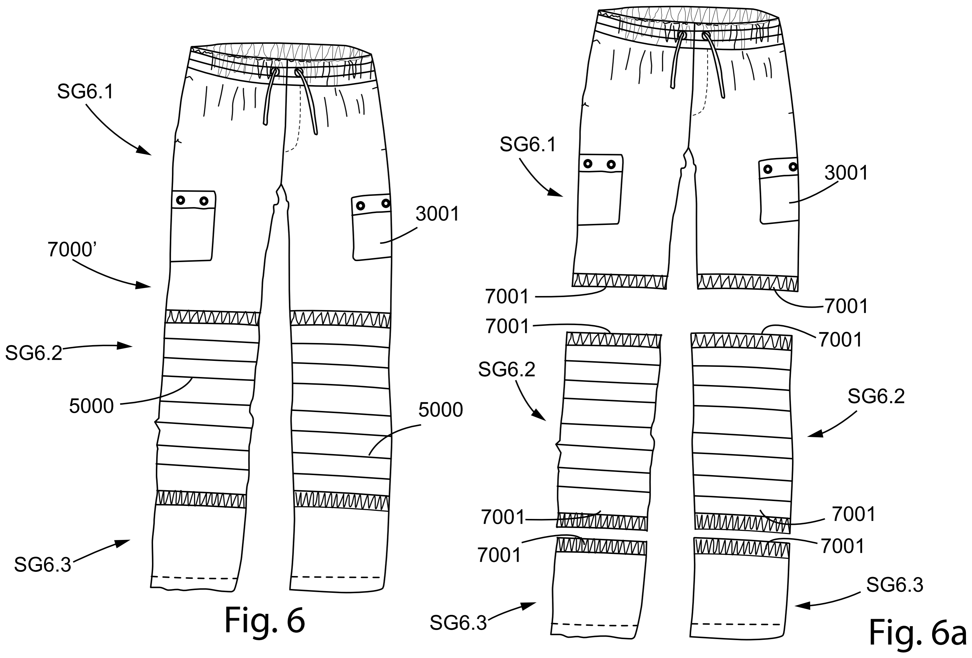

Figure **6**A shows an exploded front view of the therapeutical device of Figure **6****;**

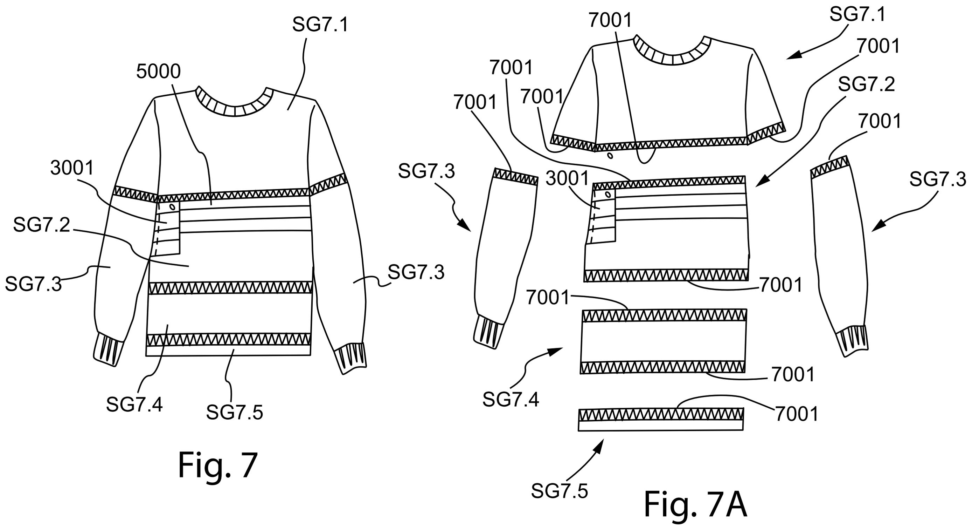

Figure **7** shows a front view of a therapeutical device according to a third embodiment of the present invention, whose anatomical support has the shape of a sweatshirt or pullover;
Figure **7**A shows an exploded front view of the therapeutical device of Figure **7****;**

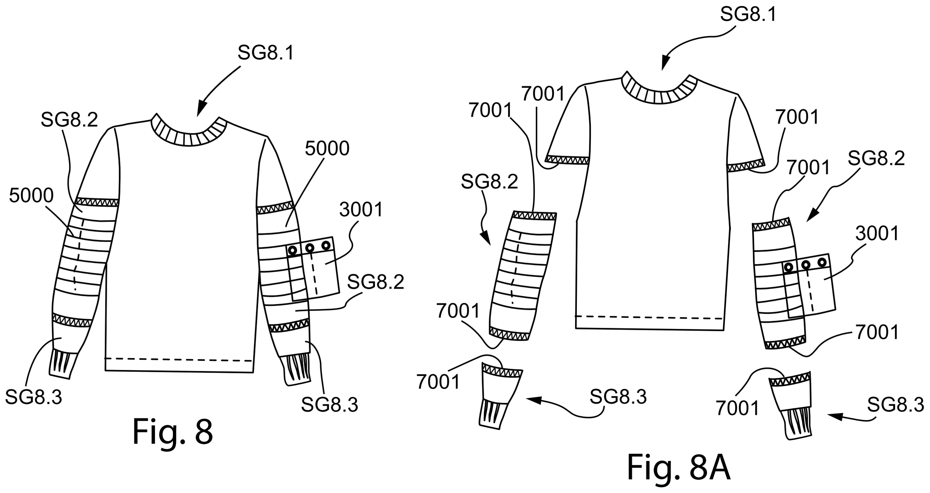

Figure **8** shows a front view of a therapeutical device according to a fourth embodiment of the present invention, whose anatomical support has the shape of a sweatshirt or pullover;
Figure **8**A shows an exploded front view of the therapeutical device of Figure **8****;**

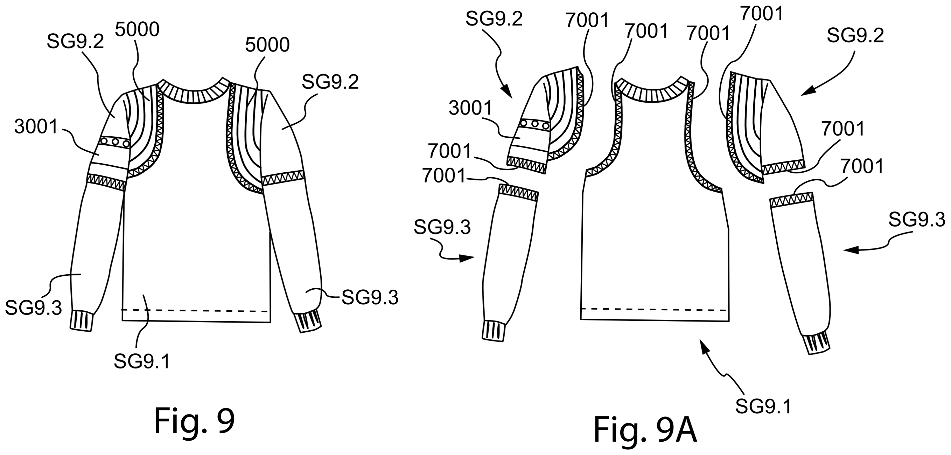

Figure **9** shows a front view of a therapeutical device according to a fifth embodiment of the present invention, whose anatomical support has the shape of a sweatshirt or pullover;
Figure **9**A shows an exploded front view of the therapeutical device of Figure **9****;**

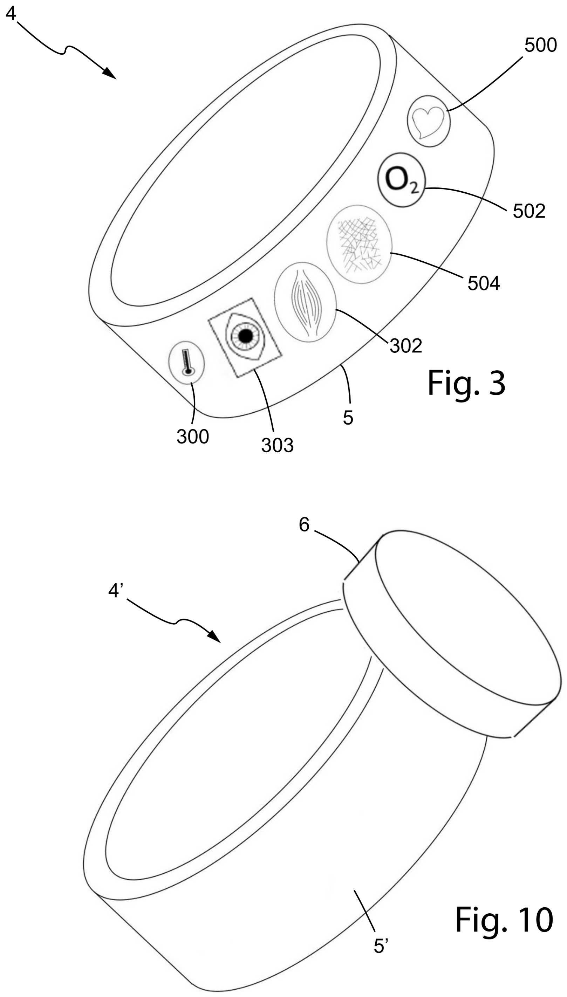

Figure **10** shows a perspective view of a calibration unit of an inflammation detection system according to a second particular embodiment of the present invention;

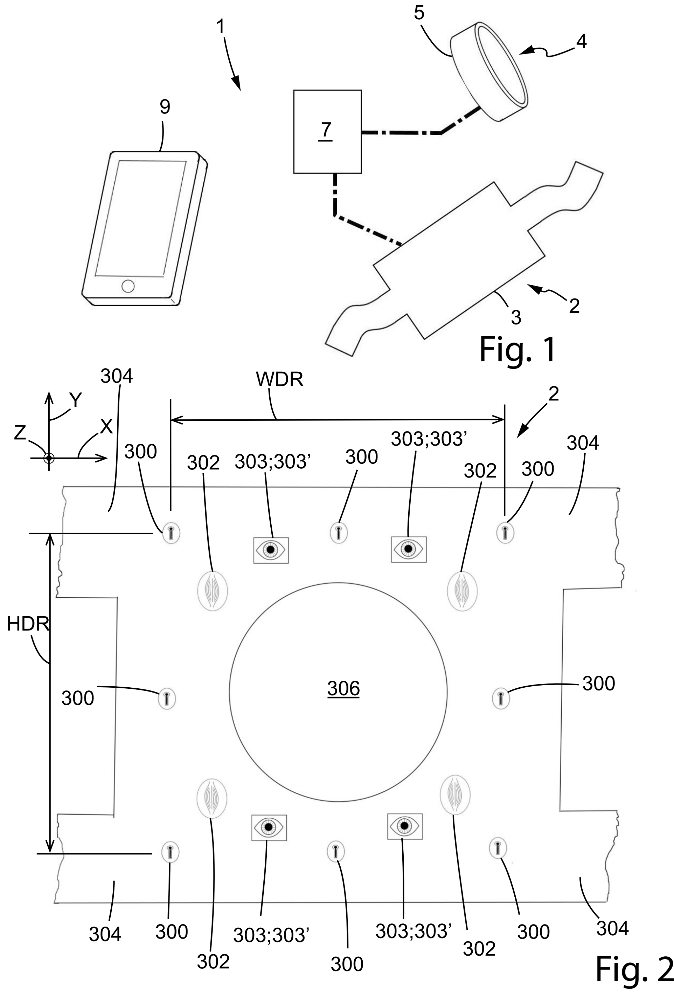

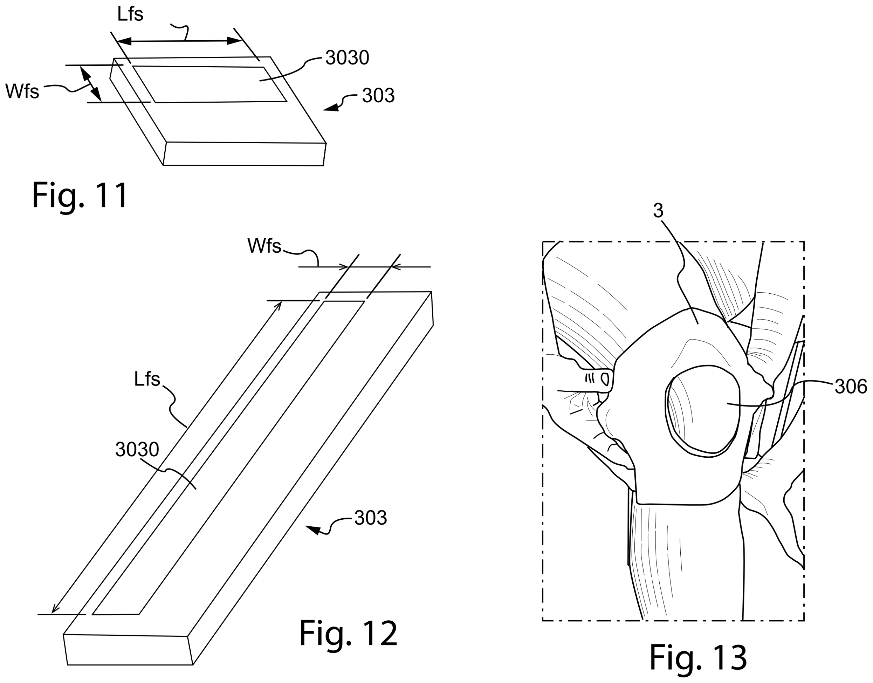

Figure **11** shows a perspective view of a first example of optical sensor usable in the detection unit of Figure **2****;**
Figure **12** shows a perspective view of a second example of optical sensor usable in the detection unit of Figure **2****;**
Figure **13** shows a perspective view of a patient wearing on a knee the detection unit of Figure **2****;**

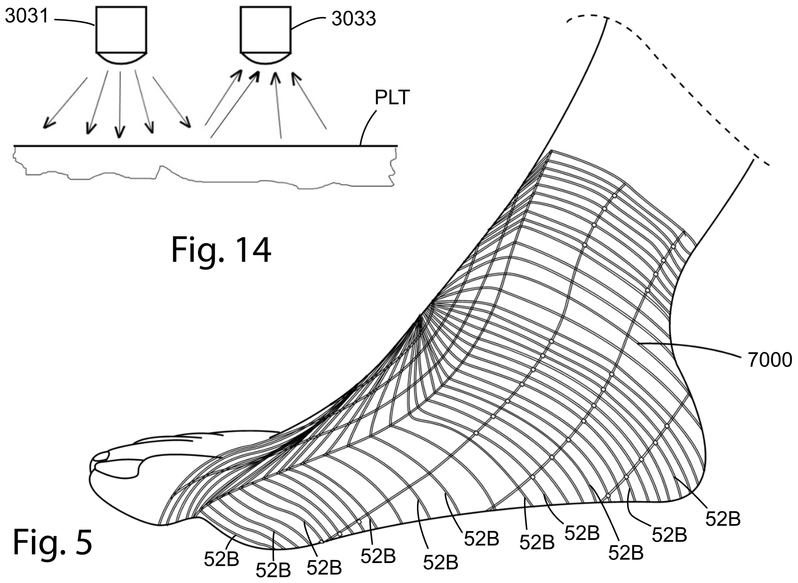

Figure **14** shows a functional scheme of a third example of optical sensor usable in the detection unit of Figure **2****;**

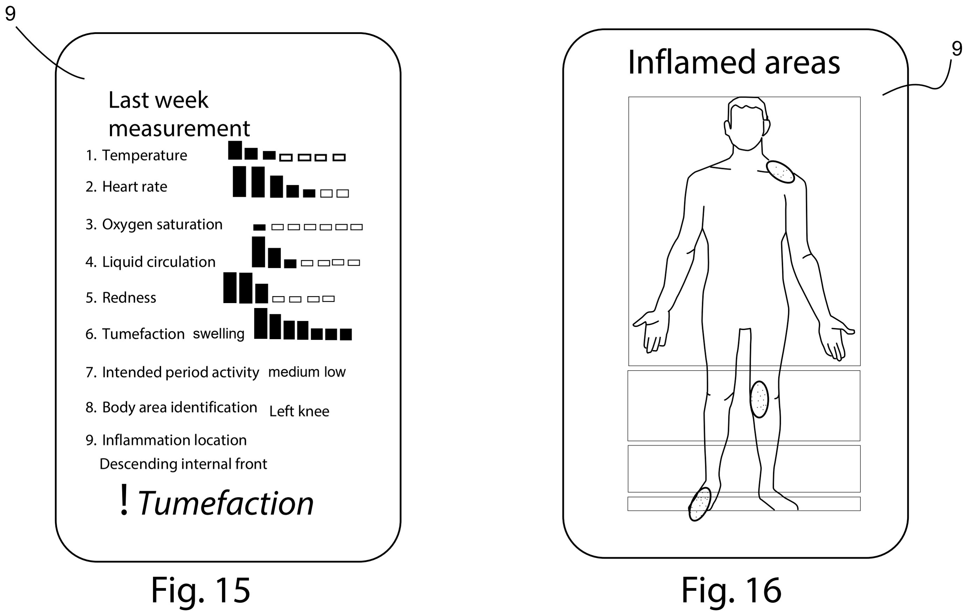

Figure **15** shows a first screen displayable by the graphic interface of the system of Figure **1****;**
Figure **16** shows a second screen displayable by the graphic interface of the system of Figure **1****;**

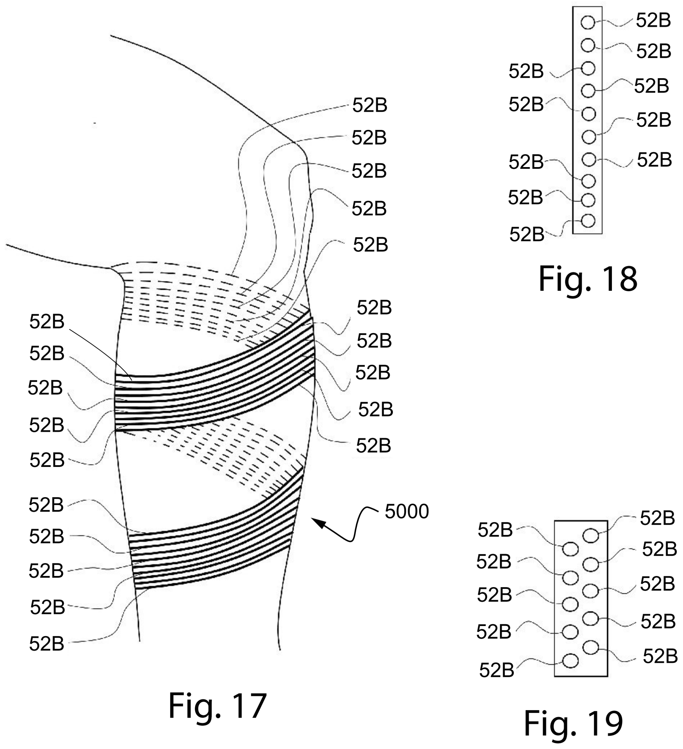

Figure **17** shows a perspective view of a leg of a patient or other user wearing an electromagnetic distribution element of a therapeutical device according to a sixth embodiment of the present invention;
Figure **18** shows a partially sectional view of a portion of the electromagnetic distribution element of Figure **17****,** according to an ideal section plane perpendicular to the space direction along which the wires forming the coils of the electromagnetic distribution element extend;
Figure **19** shows a partially sectional view of a portion of the electromagnetic distribution element of Figure **17****,** according to an ideal section plane perpendicular to the space direction along which the wires forming the coils of the electromagnetic distribution element extend, in which electromagnetic distribution element the electrically conductive wires form a thin-strip distribution different from that of Figure **18****;**

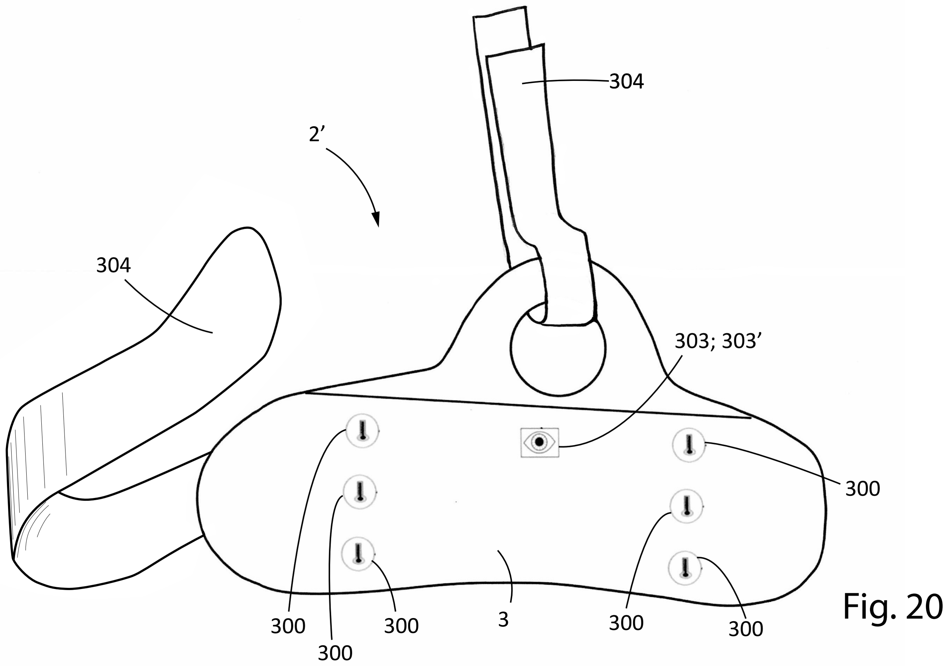

Figure **20** shows a front view of a second possible variant of the detection unit of the system of Figure **1****.**

### Detailed description

Figures **1-3** relate to a system for detecting inflammation of an anatomical part of a user, whether human or animal, according to a first embodiment of the invention and indicated as a whole by overall reference **1.**

Such anatomical part can be, for example, a shoulder, an arm, a forearm, an elbow, a wrist, one or more fingers of a hand or foot, a hand, a foot, a leg, a thigh, a knee, a calf, an ankle, a foot, an arm, a forearm, an elbow, a shoulder, the neck, the trunk, the chest, the back, the abdomen, the lumbar area of the back, a hip, the pelvis, or the area at or in proximity to one or more vertebrae.

The detection system **1** comprises a plurality of sensors of at least two types, wherein said types are selected from the following list: a body temperature sensor **300,** a muscular contracture and/or contraction sensor **302,** an electromyographic sensor, which can optionally make the above-mentioned muscular contracture sensor, a heart rate sensor, a blood haemoglobin oxygen saturation sensor, a skin perspiration sensor, a skin electrical conductance or electrodermal activity sensor, an optical sensor **303, 303'.**

The assembly of said sensors is programmed or otherwise adapted to determine the level of inflammation of an anatomical part based on the detections of one or more of the following body parameters of the anatomical part: skin temperature, redness, or more generally colour, tumefaction, anomalous liquid infiltration, swelling, pain, pathological or otherwise anomalous muscular contracture, heart rate, blood oxygenation, sweating.

Said body temperature sensor **300** is preferably adapted to provide a measurement in degrees Celsius, Fahrenheit, or Kelvin of the temperature of the anatomical part of interest.

Said body temperature sensor **300** is preferably adapted to detect the difference between the temperature of the anatomical part of interest and a reference temperature.

One or more of said sensors can be adapted to detect tumefaction, liquid circulation or spill, swelling and/or sweating of the anatomical part of interest, by detecting, for example, an electrical conductance or electrodermal activity, preferably an electrical conductance detected between two electrodes applied to the skin of the patient or otherwise of the user of the system **1.**

The system **1** can be adapted to express or otherwise provide a measurement, for example in microsiemens, of said electrical conductance: the conductance measurements performed on a human or animal body for the purpose of detecting tumefaction, liquid circulation or spill, swelling and/or sweating are not significantly affected by the distance between the two measurement electrodes applied to the human or animal body under examination, at least for the purposes of detecting possible inflammation states.

Said electromyographic sensor **302** can comprise two or more surface electrodes adapted to be applied to the skin of the patient and to detect the surface electromyographic signal.

One or more of said sensors **303, 303'** can be adapted to detect skin redness or, more generally, colour of the anatomical part of interest.

For this purpose, one or more of said sensors **303'** can each comprise a light emitter **3031** and a light receiver **3033** (Figure **14**)**.**

The light emitter **3031** is adapted to irradiate a skin portion PLT of the patient or user with electromagnetic radiation in the visible band to the naked eye or, more preferably, in the infrared band.

The light receiver **3033** is adapted to detect electromagnetic radiation, preferably in the visible band and/or infrared band, reflected by the skin portion irradiated by the emitter **3031.**

Advantageously, the sensor **303'** can be provided with multiple light emitters **3031,** for example one adapted to emit infrared radiation and another to emit visible light, preferably red light.

Infrared light emitters **3031** and receivers **3033** can advantageously operate even when shielded by a protective and containing fabric that preferably encloses each sensor **303'** and possibly other sensors **300, 302, 303** of the detection unit **2, 2'** described further below, so as to be much less affected by ambient light.

The combination of an optical emitter **3031** and receiver **3033,** or other optical sensors **303',** can be adapted to detect the skin colour of the patient or user by detecting the frequency or wavelength of the skin colour itself.

Some advantages of the optical sensor **303'** using LEDs or pairs of different light emitters and receivers, or other electromagnetic radiation close to the visible band, include small size, very low energy consumption, and the ability to also measure skin reflectance, which is an index of anomalous liquid infiltration, tumefaction, and swelling: indeed, the skin of a tumefaction area or affected by anomalous liquid spill is shinier and therefore has greater reflectance.

Still for such purpose, one or more of said sensors can comprise an image sensor **303** adapted to acquire images of the skin of the anatomical part of interest and to which the sensor **303** is facing or otherwise directed, or another optical sensor **303** configured for detecting the skin coloration of such anatomical part.

The image sensor **303** can be configured for acquiring an image, for example in digital format.

For this purpose, the image sensor **303** can be of the type used in digital cameras or camcorders and comprise, for example, a semiconductor integrated circuit of the charge-coupled device (CCD) type, the active-pixel sensor (APS) type, or the complementary APS MOS type (CMOS), also commonly referred to simply as a "CMOS sensor".

The system **1** is preferably configured for deriving a colour number from the digital image of the skin acquired by the image sensor **303** or other optical sensor **303.**

Such colour number can be, for example, a pure number advantageously obtained from the chromatic coordinates of the **1**-LAB colour spaces (Luminance + A + B), preferably combined with the HSB (Hue Saturation Brightness) colour space, or from the CIELAB, CIE XYZ, CMYK, HSV, HSL, Adobe RGB, sRGB, ISO RGB, Extended ISO RGB, Apple RGB, or other RGB-based colour spaces.

Each image sensor **303,** or more generally each optical sensor **303,** is provided with a photosensitive surface **3030** adapted to convert into electrical signals the light that irradiates it.

The plan shape of said photosensitive surface **3030** can be substantially square or rectangular and relatively short (Figure **11**), or relatively elongated (Figure **12**), for example with a ratio between length **Lfs** and width **Wfs** equal to or greater than **1.5** times, or equal to or greater than **2** times, **3** times, **5** times, **10** times, or **20** times.

The **Lfs/Wfs** ratio can be for example between **1** and **50** times or between **1** and **100** times.

Pain can be detected by one or more of the previously described sensors, possibly by suitable algorithms implementing predictive models, for example by deducing a pain state from a particular increase in heart rate and/or sweating, and/or by a questionnaire, for example a self-assessment questionnaire, filled out by the patient/user of the detection system **1.**

For this purpose, the detection system **1** is advantageously programmed or otherwise adapted to present, for example by the graphic and/or acoustic interface **9** described below, such an interactive questionnaire to the patient/user to be filled out, and to acquire and process the responses entered by the patient/user in such questionnaire.

For this purpose, the detection system **1** can be programmed or otherwise adapted to present such interactive questionnaire to the patient/user to be filled out before detecting one or more of the above-mentioned body parameters by the above-mentioned sensors **300, 302, 303, 303',** and/or to acquire the responses entered by the patient/user in such questionnaire before detecting one or more of the above-mentioned body parameters by the above-mentioned sensors **300, 302, 303, 303'** .

As a reference value for said temperature, redness, tumefaction, anomalous liquid infiltration, swelling, pain, pathological or otherwise anomalous muscular contracture, heart rate, blood oxygenation, sweating, and redness to be measured, the value detected or otherwise determined from a reference anatomical part is preferably used, which reference anatomical part can in turn be the same anatomical part of interest or a different one, under conditions considered to be free of inflammation or in a low level of inflammation.

More preferably, such reference temperature, redness, tumefaction, liquid infiltration, swelling, pain, muscular contracture, heart rate, blood oxygenation, sweating, or conductance is respectively the corresponding body parameter, for example, of a wrist, ankle, finger, area at or in proximity to the lower back, or a sacroiliac joint being non-inflamed or only slightly inflamed, of the subject for whom the degree of inflammation is to be detected.

The detection system **1** is preferably programmed or adapted to determine an acute inflammation state based on the detections of one or more, and more preferably all, and possibly only all, of the following body parameters: temperature, tumefaction, liquid circulation, swelling, sweating, redness, haemoglobin oxygen saturation, muscular contracture, sweating, heart rate.

The detection system **1** can be, for example, programmed or adapted to determine an acute inflammation state based on the detections of temperature and at least one other body parameter selected from pain, tumefaction, liquid circulation, swelling, sweating, redness, sweating, skin electrical conductance or electrodermal activity.

The detection system **1** is preferably programmed or adapted to determine a subacute inflammation state based on the detections of one or more, and more preferably all, and possibly only all, of the following body parameters: skin electrical conductance or electrodermal activity, for example, muscular contracture, pain.

More preferably, the detection system **1** is programmed or adapted to determine a subacute inflammation state if at least the skin electrical conductance or electrodermal activity, for example, muscular contracture, of the examined part exceeds normal limits for a period longer than three months and equal to or shorter than nine months.

The detection system **1** is preferably programmed or adapted to determine a chronic inflammation state at least based on the detection of muscular contracture.

The detection system **1** is preferably programmed or adapted to determine a chronic inflammation state at least based on the detection of skin electrical conductance or electrodermal activity, for example, muscular contracture, and pain.

More preferably, the detection system **1** is preferably programmed or adapted to determine a chronic inflammation state if at least temperature and skin electrical conductance or electrodermal activity, for example, muscular contracture, and/or pain in the examined part exceed the normal limits for a period longer than nine months.

More generally, the detection system **1** can preferably be programmed or adapted to determine an acute, subacute, or chronic inflammation state if one or more of the body parameters it detects have anomalous values, respectively, for a period equal to or shorter than three months, between three and nine months, and longer than nine months.

The system **1** preferably comprises an anatomical interface **3, 3', 5** adapted to fix two or more of said sensors to the user's body, and which can form, for example, one or more of the following elements: a patch of a membrane or other relatively foldable material (Figures **1, 2**), a bandage, a strap, a bracelet, a sleeve, a sock to be worn for example on a foot, a glove, a short- or long-sleeved t-shirt, a tank top, a shirt, a gilet, a bib, underwear, short or long pants, a skirt, a hat, a cap, a membrane, a more or less rigid shell, a flat or non-planar plate, a wristband, a knee brace, an elbow brace, an ankle brace, a finger ring or a finger cot.

More generally and preferably, the anatomical interface **3, 3', 5** is adapted to be worn, i.e., it is wearable.

For example, when forming a patch or a strap, the anatomical interface **3, 3', 5** can comprise, for example, ties **304,** belts, buttons, buckles, pins, zippers, or hook-and-loop fasteners that allow the interface **3, 3', 5** to be fixed to the user's body (Figure **2**).

In a more or less central area, the patch or strap formed by the anatomical interface **3, 3', 5** can form a hole or opening **306** adapted to be crossed and/or allow the escape, for example, from the wrapping formed by the patch or strap, or more generally from the anatomical interface, of a knee, an elbow, a finger, or other appropriate anatomical parts (Figures **2, 13),** also serving to properly centre and position the anatomical interface with respect to the anatomical part to be treated.

When forming, for example, a glove, a sock, a sleeve, or a pants leg, the anatomical interface **3, 3', 5,** instead of a positioning hole or other opening **306,** can be provided with a slot, one or more ties, or markers that allow correct positioning of the anatomical part, referring for example to a finger, a wrist, a knee, elbow, joint, articulation, prominence, or bone end.

The system **1** more preferably comprises:
- at least one detection unit **2, 2',** in turn comprising the measurement anatomical interface **3, 3';** and
- at least one calibration unit **4,** in turn comprising the calibration anatomical interface **5.**
The detection unit **2** of Figure **2** is particularly suitable for detecting inflammation, for example, in shoulder, knee, arm, and hip joints.
The detection unit **2'** of Figure **20** is particularly suitable for detecting inflammation, for example, in the wrist, ankle, and/or sacroiliac area joints; for such purpose, the detection unit **2'** can be free of electromyographic and/or contracture sensors.

The measurement anatomical interface **3, 3'** is configured for being fixed to a first anatomical part of the user's body, and the sensors of the measurement anatomical interface are configured for detecting one or more body parameters of the first anatomical part.

The calibration anatomical interface **5** is configured for being fixed to a second anatomical part of the user's body, and the sensors of the calibration anatomical interface are configured for detecting one or more body parameters of the second anatomical part.

The system **1** is preferably adapted to calibrate the detections of the sensors fixed to the measurement anatomical interface **3, 3'** based on the detections from the sensors fixed to the calibration anatomical interface **5.**

Said sensors are preferably fixed to the first **3** and/or the calibration anatomical interface **5.**

Preferably, the calibration anatomical interface **5** comprises a bracelet, a wristband, an ankle brace, a glove, a sock, a sleeve configured for containing an arm, forearm, calf, thigh or leg, a collar, a strap, or other patch configured for being fixed to an anatomical part such as at least a part of the torso, pelvis, groin, abdomen, belly, back, one or more shoulders of the patient, the lower back, or in proximity to or at a sacroiliac joint.

When it is a wristband, ankle brace, or more generally adapted to be fixed to an anatomical part lacking muscles, the calibration anatomical interface **5** can be free of myographic sensors **302.**

Preferably, the measurement anatomical interface **3, 3',** and more generally the detection unit **2, 2'** comprises sensors of at least two types selected from a temperature sensor **300,** an electromyographic **302** and/or muscular contracture sensor **302,** and an optical sensor **303** (Figure **2**).

For this purpose, the measurement anatomical interface **3, 3',** and more generally the detection unit **2, 2',** can comprise a number of sensors of each type between two and one hundred, or between three and thirty, between three and twenty, between four and nine, or between four and seven, for example, a number of body temperature sensors between two and thirty and/or a number of electromyographic and/or muscular contracture sensors **302** between two and thirty.

Preferably, the calibration anatomical interface **5,** and more generally the calibration unit **4,** comprises sensors belonging to at least two of the following types: a temperature sensor **300,** a heart rate sensor **500,** a blood haemoglobin oxygen saturation sensor **502,** a skin perspiration sensor **504,** a skin electrical conductance or electrodermal activity sensor **504,** an electromyographic and/or muscular contracture and/or sweating sensor **302,** an image sensor **303** or more generally an optical sensor **303, 303'**, and an accelerometer (Figure **3**).

The blood haemoglobin oxygen saturation sensor **502** and/or the heart rate sensor **500** of the calibration anatomical interface **5,** and more generally of calibration unit **4,** can be adapted to be fixed to a finger of the patient or user.

Advantageously, the sensors of the calibration anatomical interface **5** are configured for detecting one or more body parameters substantially of the same type as the body parameters detected by the sensors of the measurement anatomical interface **3, 3'.**

The rows of Table **9** below provide some examples of what is meant in the present description by "body parameters of the same type".

**- Table 9 -**

| **Sensor of the detection unit 2, 2', or fixed to the measurement anatomical interface 3, 3'** | **Sensor of the same type of the calibration unit 4, or fixed to the calibration anatomical interface 5** |
|---|---|
| temperature sensor | temperature sensor |
| heart rate sensor | heart rate sensor |
| blood haemoglobin oxygen saturation sensor | blood haemoglobin oxygen saturation sensor |
| skin perspiration sensor | skin perspiration sensor |
| skin electrical conductance sensor | skin electrical conductance sensor |
| electromyographic and/or muscular contracture and/or sweating sensor | electromyographic and/or muscular contracture and/or sweating sensor |
| electrodermal activity sensor | electrodermal activity sensor |
| skin colour sensor | skin colour sensor |

Advantageously, the system **1** is configured for calibrating the detections of the sensors fixed to the measurement anatomical interface **3, 3',** or more generally of the detection unit **2, 2',** by deriving an index of absence of inflammation or of low or otherwise negligible inflammation from the detections of one or more body parameters acquired by one or more sensors fixed to the calibration anatomical interface **5,** or more generally by the calibration unit **4.** For this purpose, the system **1** can be adapted to take as such an index the above-mentioned detections of one or more body parameters acquired by one or more sensors fixed to the calibration anatomical interface **5,** or more generally by the calibration unit **4.**

For this purpose, the system **1** can be adapted to derive an indication that at the k-th instant, the anatomical part to which the calibration anatomical interface **5** is fixed is not substantially inflamed, from the value **Var_Par(**i**)_Tar(**k) of a certain i-th body parameter, such as temperature, heart rate, skin perspiration, skin electrical conductance, muscular contracture and/or sweating, electrodermal activity, skin colour, detected at a certain k-th instant by one of the sensors of the calibration anatomical interface **5;** for this purpose, the value **Var_Par(**i**)_Tar (**k) can be taken as an indication that at the k-th instant, the anatomical part to which the calibration anatomical interface **5** is fixed is not substantially inflamed.

Also in such case, the system **1** can be adapted, for example, to determine the degree of inflammation, for example, local inflammation of a first anatomical part, based on the difference between **a)** the detections of at least one body parameter acquired by the detection unit **2, 2'** on the first anatomical part of the user's body; and b) the detections of the same body parameter acquired by the calibration unit **4** on the second anatomical part of the user's body.

In such case, the system **1** can be configured, for example, for subtracting the value **Var_Par(**i**)_Tar(**k) from the value **Var_Par(**i**)_ Ril(**k) of the same i-th body parameter detected at the same k-th instant by one of the sensors of the measurement anatomical interface **3, 3'.**

In other words, and more generally, the system **1** can be adapted, for example, to take the detections from one or more sensors of the calibration anatomical interface **5** as a zero value for the detections from sensors of the same type on the measurement anatomical interface **3, 3'** at each predetermined k-th instant.

The above calibration process is preferably performed to calibrate the detections from a plurality of sensors of the detection unit **2, 2',** by subtracting, for example: a) the detections **Var_Par(1)_Tar(**k), **Var_Par(2)_Tar (**k) ... **Var_Par(**i)**_Tar(**k) ... **Var_Par(**N)**_Tar(**k) of a number N of sensors, and/or relating to body parameters **1, 2,** ... i... N-th, acquired by the calibration unit **4** on a second anatomical part; from b) the detections **Var_Par(1**)_**Ril(**k), **Var_Par(2**)**_Ril(**k) ... **Var_Par(**i**)_ Ril(**k) ... **Var_Par(**N)**_Ril(**k) of a corresponding number N of sensors, and/or relating to **1, 2,** ... i... N corresponding body parameters, acquired by the detection unit **2, 2'** on a first anatomical part at the same k-th instant, where i, k, and N are integers equal to or greater than **1.**

In an alternative embodiment, the system **1** can be adapted to subtract: c) the value **Var_Par(**i**)_Tar(**h), acquired at the h-th instant by at least one sensor of the calibration anatomical interface **5** and relating to a body parameter detected on a first anatomical part; from d) the value **Var_Par(**i)**_Ril_(**k), acquired at the k-th instant by at least one sensor of the measurement anatomical interface **3, 3'** and relating to the same body parameter, but detected on a second anatomical part, where h and k are positive integers and k is equal to or greater than h.

More generally, the above calibration procedure can be performed to calibrate the detections of a plurality of sensors of the detection unit **2, 2',** for example by subtracting: **a)** the detections **Var_Par(1)_Tar(**h), **Var_Par(2)_Tar (**h) ... **Var_Par(**i)**_Tar(**h) ... **Var_Par(**N)**_Tar(**h) from a number N of sensors, and/or relating to body parameters **1, 2,** ... i... N-th, acquired at the h-th instant by the calibration unit **4,** or in any case by the sensors fixed to the calibration anatomical interface **5,** on a second anatomical part; from **b)** the detections **Var_Par(1**)_**Ril(**k), **Var_Par(2**)**_Ril(**k) ... **Var_Par(**i**)_Ril_(**k) ... **Var_Par(**N)**_Ril(**k) from a corresponding number N of sensors, and/or relating to **1, 2,** ... i... N corresponding body parameters, acquired at the k-th instant by the detection unit **2, 2',** or otherwise by the sensors fixed to the measurement anatomical interface **3, 3',** on a first anatomical part, where i, h, k, and N are integers equal to or greater than **1** and k is equal to or greater than h.

The fact that index k is greater than h can imply, among other things, the following use procedure:
- at the instant h, k = **0,** acquiring by the calibration unit one or more values **Var_Par(1)_Tar(**0), **Var_Par(2)_Tar (0**) ... **Var_Par(**i)**_Tar(**0) ... **Var_Par(N)_Tar(**0), relating to a second anatomical part presumed to be non-inflamed;
- determining the degree of inflammation of a first anatomical part based on the differences between the values **Var_Par(**1)**_Ril(**k), **Var_Par (2)_Ril(**k), ... **Var_Par(**i**)_Ril_(**k), ... **Var_Par(**N)_**Ril(**k), continuously acquired by the measurement unit **2** at subsequent instants k = **2, 3, 4...** M, and the values **Var_Par(1)_Tar(0**), **Var_Par(2)_Tar (0**) ... **Var_Par(**i)**_Tar(0**) ... **Var_Par(**N)**_Tar(0**)**.**

This latter use procedure allows to perform a single initial calibration phase by the calibration unit **4,** followed by a phase of continuous measurements, lasting more or less over time, on the first anatomical part by the detection unit **3,** simplifying the use of the system **1.**

The above calibration processes and expedients generally allow for more precise and sensitive detections of inflammation by the detection unit **2, 2',** adapting them, for example, at each instant, to the current physiological conditions of the specific patient, such conditions being detected by the calibration unit **4** only in an initial calibration phase or continuously during the operation of the detection unit **2, 2'.**

Advantageously, the sensors of the measurement anatomical interface **3, 3'** are arranged so as not to be aligned along a single straight line, and are preferably aligned along two or more straight lines that are parallel, perpendicular, or transverse to each other, or even to form a preferably two-dimensional distribution, at least when the measurement anatomical interface **3, 3'** is developed on a flat surface.

Such two-dimensional distribution has a maximum width **WDR** and/or height **HDR** preferably between **4.0** and **80.0** centimetres, for example between **8.0** and **40.0** centimetres, or between **16.0** and **30.0** centimetres.

The sensors of the measurement anatomical interface **3, 3'** can also be arranged, for example, in a staggered configuration and/or at the vertices of a grid with triangular, square, or rectangular meshes; they can be aligned along rows and columns, where rows and columns are perpendicular or more generally transverse to each other.

The maximum distance between two different sensors of the measurement anatomical interface **3, 3'**, whether they are sensors of the same or different types, and adapted to detect two body parameters of the same or different type, where in the latter case, one sensor detects a temperature and the other the skin colour or liquid presence in the tissues, is preferably equal to or lower than **40.0** centimetres, or **30.0** centimetres, **20.0** centimetres, **10.0** centimetres, **5.0** centimetres, or **2.0** centimetres.

The maximum distance between two different sensors of the calibration anatomical interface **5,** whether they are sensors of the same or different types, and adapted to detect two body parameters of the same or different type, where in the latter case, one sensor detects a temperature and the other the skin colour or liquid presence in the tissues, is preferably equal to or lower than **40.0** centimetres, or **30.0** centimetres, **20.0** centimetres, **10.0** centimetres, **5.0** centimetres, or **2.0** centimetres.

More preferably, the maximum distance between two different sensors of the calibration anatomical interface **5** is substantially lower than the maximum distance between two different sensors of the measurement anatomical interface **3, 3',** and, for example, it is half, one-third, one-fourth, one-sixth, or one-eighth of the maximum distance between two different sensors of the measurement anatomical interface **3, 3'.**

Such relatively short distances between the sensors of the detection **2, 2'** and calibration **4** units are particularly advantageous and effective for detecting inflammation of single anatomical parts, that is, local inflammations rather than systemic inflammations, which by definition affect the entire body.

Advantageously, the detection unit **2, 2'** comprises at least two sensors **300, 302** of the same type for each of at least two of said types, and the system 1 is advantageously programmed or otherwise adapted to determine a gradient, trend, or other variation, as a function of the space, of the body parameter determined by said at least two sensors **300, 302** of the same type (Figure **2**).

Preferably, the calibration anatomical interface **5,** and more generally the calibration unit **4,** comprises sensors belonging to at least two of the following types: a temperature sensor **300,** a heart rate sensor **500,** a blood oxygen saturation sensor **502,** a skin perspiration sensor **504,** a skin conductance sensor **504,** an electromyographic and/or muscular contracture and/or sweating sensor **302** (Figure **3**)**.**

In general, the system **1** can use, among other things, the detections of heart rate, blood oxygen saturation, and temperature as instantaneous indicators of a subject's general health status and, if necessary, as previously mentioned, as reference values for a state of presence or absence of systemic inflammation.

Two or more temperature **300,** heart rate **500,** blood oxygen saturation **502,** skin perspiration **504,** skin electrical conductance **504,** or electrodermal activity, sensors, and/or electromyographic and/or muscular contracture and/or sweating sensors **302** can be distributed over the surface of the calibration unit **4** configured for touching the patient's body or otherwise facing the patient's body, at greater or lower distances from each other, for example, they can be distributed substantially uniformly across the surface of calibration unit **4** configured for touching the patient's body or otherwise facing the patient's body (Figure **3**).

Alternatively, two or more temperature **300,** heart rate **500,** blood oxygen saturation **502,** skin perspiration **504,** skin electrical conductance **504** sensors, and/or electromyographic and/or muscular contracture and/or sweating sensors **302** can be housed or otherwise arranged within a case, box, or other enclosure **6** having overall dimensions smaller than those of anatomical interface **5** (Figure **10**); in such case, the case or box **6** can resemble, for example, the case of a wristwatch.

The detection of heart rate and body temperature on a wrist or another reference anatomical part by the calibration unit **4** is advantageously used as a general index and reference for assessing the state and general deviation of the single subject from "normal" threshold values indicated by the scientific literature.

More advantageously, the system **1** is advantageously programmed or otherwise adapted to determine a gradient or other variation along at least two space directions **X, Y** of the body parameter determined by said at least two sensors of the same type, where said at least two space directions **X, Y** are substantially orthogonal or transverse to each other (Figure **2**).

For example, as shown in Figure **2****,** the detection unit **2, 2'** is configured for detecting:
- a gradient, or other trend or variation, of temperature along direction **X** and/or direction **Y** by the temperature sensors **300;** and
- a gradient, or other trend or variation, of muscular contracture and/or muscular contraction along direction **X** and/or direction **Y** by the muscular contracture and/or electromyographic sensors **302.**

Two or more of said sensors of the same type, for example, two or more temperature sensors, or two or more electromyographic or optical sensors, can be fixed to the anatomical interface **3, 3', 5** at a distance, for example, vertical or horizontal, from each other preferably between **3** and **120** millimetres, or between **7** and **60** millimetres, between **10** and **30** millimetres, or between **15** and **25** millimetres, and more preferably between **25** and **40** millimetres, between **28** and **35** millimetres, between **35** and **45** millimetres, or between **40** and **53** millimetres.

Advantageously, three or more of said sensors of the same type are fixed to the anatomical interface **3** aligned along one or more rows, for example horizontal and/or vertical.

Two or more of such rows can, for example, be substantially parallel or longitudinal to each other.

Two or more of such rows can, for example, be substantially perpendicular or transverse to each other.

Such sensor alignments in rows, optionally longitudinal and/or transverse to each other, allow the measurement of the above-mentioned gradients or other trends and variations of one or more body parameters of the anatomical part of interest, providing greater insights into the possible inflammation state of the anatomical part.

As an alternative to arranging a plurality of sensors of the same type in rows, the detection unit **2, 2'** can also comprise only a single sensor of each type, and such a sensor has a sensitive element having a length and/or width, for example, between **4.0** and **80.0** centimetres, or between **8.0** and **40** centimetres or between **16.0** and **30.0** centimetres, so that the sensitive element has a sufficiently large extension to independently detect one of the above-mentioned gradients, trends, or other variations of a body parameter.

The plurality of temperature sensors **300,** or the single relatively extended temperature sensor, is advantageously configured for detecting gradients or other temperature distributions as a function of the space with a precision preferably equal to or lower than **0.5°**C, more preferably equal to or lower than **0.2°C, 0.05°C, 0.03°C, 0.02°C or 0.16°C.**

Such precisions allow the detection of temperature distributions with adequate precision, repeatability, and reliability.

The system **1** is preferably programmed or otherwise adapted to detect a situation of inflammation when tumefaction, liquid circulation or spill, swelling, sweating, skin coloration, muscular contracture, temperature, or other body parameters at or in proximity to an anatomical part of interest are anomalous in magnitude.

For this purpose, the system **1** is preferably programmed or otherwise adapted to implement the following process:
**S1.1**) determining an admissibility range for one or more body parameters, such as, for example, tumefaction, liquid circulation or spill, swelling, sweating, skin coloration, skin electrical conductance or electrodermal activity, muscular contracture, or temperature, wherein such admissibility range can be one-dimensional or, as in the case of skin colour determination in colour spaces, also multidimensional;
**S1.2**) signalling an anomalous intensity or magnitude of said one or more body parameters of the anatomical part of interest when the system **1** detects values of said one or more body parameters outside the respective admissibility range.

The admissibility range indicates the range within which a body parameter does not indicate, at least not with sufficient reliability or significance, an inflammation state, and can be a limited, i.e., between a lower and an upper threshold, or unlimited range, for example, delimited only by a lower or upper threshold.

Preferably, an admissibility range of a body parameter contains its normal value(s) corresponding to the absence of inflammation.

An admissibility range of a body parameter preferably comprises a safety margin and extends beyond the normal values of the body parameter in question.

In operation **S1.2**), values of said one or more body parameters can be detected outside the respective admissibility range, for example, if they are equal to or greater than a predetermined upper threshold and/or lower than a predetermined lower threshold.

In operation **S1.2**):
- the measured temperature value at or in proximity to the anatomical part of interest can be detected as outside its normal range, for example, if it is at least **0.5**°C higher than the upper admissibility threshold, for example if the anatomical part of interest is a hand or foot finger, wrist, elbow, knee, or ankle; or it can be considered outside its normal range if it exceeds the upper admissibility threshold by at least **0.8-1.0°C,** for example when the anatomical part of interest is the neck, shoulder, or one or more vertebrae;
- the value of tumefaction, liquid circulation or spill, swelling and/or sweating at or in proximity to the anatomical part of interest can be detected as outside its normal range, for example if it is at least **3.0** microsiemens lower, and more preferably at least **5.0, 7.0,** or **10.0** microsiemens lower, than the lower admissibility threshold, and/or at least **3.0** microsiemens higher, and more preferably at least **5.0, 7.0,** or **10.0** microsiemens higher, than the upper admissibility threshold;
- the skin colour at or in proximity to the anatomical part of interest can be detected as outside its normal range, for example if the wavelength of the light corresponding to the skin colour is at least **50** nanometres lower, and more preferably at least **100** or **150** nanometres lower, than the lower admissibility threshold, and/or at least **50** nanometres higher, and more preferably at least **100** or **150** nanometres higher, than the upper admissibility threshold;
- alternatively, or in combination with the above, the skin colour at or in proximity to the anatomical part of interest can be detected as outside its normal range if its point in the LAB or CIELAB colour space is located at a distance equal to or greater than a predetermined threshold from the boundaries of the admissibility area within such colour space;
- the blood haemoglobin oxygen saturation (SpO**2**) at or in proximity to the anatomical part of interest can be detected as outside its normal range, for example if the saturation percentage is at least **2.0%** lower than the lower admissibility threshold;
- the muscular contracture at or in proximity to the anatomical part of interest can be detected as outside its normal range, for example if it exceeds the upper admissibility threshold by at least **0.05** millivolts, and more preferably by at least **0.10** millivolts.

The system **1** can be programmed or otherwise adapted to indicate an inflammation state if sweating in the anatomical part of interest is outside its admissibility range, i.e., has an anomalous value, for example, when the patient or user experiences pain while trying to bend a joint inflamed due to an impact, fracture, dislocation, or other trauma.

As partially already mentioned, the system **1** is advantageously programmed or otherwise adapted to determine the admissibility range of one or more of the above-mentioned bodily parameters based on the detections of the calibration unit **4.**

The inflammation detection system **1** is advantageously programmed or otherwise adapted to determine an index of the level of inflammation of an anatomical part, for example, as previously stated, based on the detections of one or more of the following body parameters of the anatomical part of interest: temperature, redness, tumefaction, anomalous liquid infiltration, pain, pathological or otherwise anomalous muscular contracture, heart rate, blood oxygenation, sweating.

Such index of inflammation is preferably a numerical index, expressed for example in the form of positive and/or negative integers, or positive and/or negative real numbers.

The inflammation detection system **1** advantageously comprises a logic unit **7** adapted to store, i.e., save, and retain the detections of the detection unit **2, 2'** and/or of the calibration unit **4.**

The logic unit **7** can be local, i.e., adapted to be worn by the final user who uses the detection unit **2, 2'** and/or the calibration unit **4,** or a remote logic unit, i.e., not worn by the final user during operation of the detection unit **2, 2'** and/or the calibration unit **4** and, for this purpose, can be located several metres or kilometres away from the final user, being, for example, located in a remote server computer.

The logic unit **7** is advantageously programmed or adapted to process the detections of the detection unit **2, 2'** and/or of the calibration unit **4** by comparing them with past detections related to one or more final users, for example by artificial intelligence and/or machine learning methods, so as to improve the accuracy of measurements and predictions regarding the inflammation state.

Also, though not exclusively for such purpose, the logic unit **7** is advantageously programmed or adapted to monitor and examine the evolution over time of the inflammation state and the body parameters of a final user, and possibly to compare it with similar states and parameters of other final users or patients, still with the goal of significantly improving the determinations, measurements, and predictions of each detection system **1** used by each final user.

Comparing correlations between body parameters and inflammation state or degree across a large number of patients, or otherwise users, allows highly accurate automatic detections by the inflammation detection systems **1.**

The system **1** allows for early detection with notable sensitivity of even latent or otherwise poorly perceived irritation conditions by the patient, thereby allowing early identification and intervention in acute or subacute phases, and helping to prevent inflammation from becoming chronic, which is difficult to treat.

The logic unit **7** is preferably programmed or adapted to calculate or otherwise determine the above-mentioned inflammation index.

The inflammation detection system **1** advantageously comprises a graphic and/or acoustic interface **9** configured for displaying in texts or other images and/or for translating into voice messages or other coded sounds the detections of the detection unit **2, 2',** of the calibration unit **4** and/or past detections or their processing stored or generated by the above-mentioned logic unit **7,** whether this logic unit is local or remote.

For this purpose, the graphic and/or acoustic interface **9** can be configured for wirelessly communicating, for example via the Internet or other telematic networks, Wi-Fi networks, or Bluetooth or NFC communication systems.

The graphic and/or acoustic interface **9** can be provided with one or more of the following elements: a screen (for example, LCD or LED), one or more light indicators, a speaker, a buzzer.

For this purpose, the graphic and/or acoustic interface **9** can be or comprise a *smartphone,* or another handheld device, a *smartwatch,* a tablet computer, a personal computer.

The graphic interface **9** is preferably programmed or adapted to display the above-mentioned index of inflammation, for example in the form of a verbal indication, such as for example the indication "Tumefaction" in Figure **16****,** numeral and/or by a graduated scale, a bar chart (Figure **15**), the position of a needle on a dial, and/or appropriate colour coding (e.g., displaying a field or light being green with the absence of inflammation, yellow with the presence of a mild inflammation, red with the presence of a severe inflammation).

Alternatively, or in combination with the above, the acoustic interface can be programmed or adapted to communicate the index of inflammation to the final user by a voice message, for example by a synthesized human voice, and/or to display a map of the body areas with inflammation above a predetermined inflammation threshold (Figure **16**).

The inflammation detection system **1** can be advantageously used in combination with therapeutical devices adapted to treat or relieve inflammation and pain through variable electromagnetic fields, for example pulsed, such as for example the device described in Italian patent applications IT102019000006188 or IT102020000008401.

Such therapeutical devices are characterized by comprising at least one electrical generator **3000** configured for generating a variable electromagnetic field with a frequency between **2** and **120** Hertz and a maximum or peak, or effective, magnetic induction between **5** and **160** Gauss.

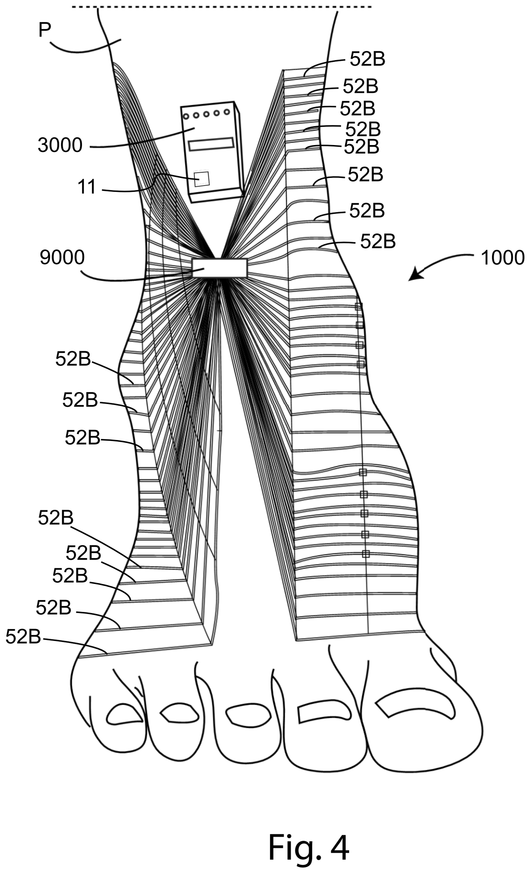

Such therapeutical device **1000** preferably comprises at least one electromagnetic distribution element **5000,** and the electrical generator **3000** is configured for powering the electromagnetic distribution element **5000** so that the latter generates said variable electromagnetic field and distributes it over a larger volume or wider surface than the volume or surface over which the electrical generator **3000** alone would distribute it (Figure **4**).

The electromagnetic distribution element **5000** can comprise one or more of the following elements: one or more electrically conductive wires **52**B (Figures **4****,** **5**), one or more electrically conductive ribbons, one or more electrically conductive sheets, a bundle, a mesh (Figures **4****,** **5**), a solenoid, one or more coils (Figures **4****, 5).**

Advantageously, the electromagnetic distribution element **5000** comprises a plurality of electrically conductive wires **52**B extending parallel or otherwise longitudinal to each other, helically wrapping around the anatomical part to be treated (Figures **17, 18**).

For this purpose, such wires **52**B preferably extend so as to form a substantially thin wire distribution, i.e., having cross-sections wider than they are thick or tall, for example, a distribution substantially having the shape of a thin bundle in which the various wires extend parallel or otherwise longitudinal to each other without substantially overlapping (Figure **18**), or arranged so that the number of overlapping layers or levels of wires is greater than the number of wires extending parallel or otherwise longitudinal to each other (Figure **19**)**.**

For this purpose, said bundle comprises a number of wires **52**B that are parallel, longitudinal, or otherwise adjacent to each other, preferably between **3** and **200** wires, and more preferably between **5** and **100** wires or between **10** and **30** wires; Figures **18** and **19****,** for example, show the cross-sections of two flat bundles made of **10** electrically conductive wires.

In this way, the electromagnetic distribution element **5000** forms a solenoid provided with a large number of coils, for example, a multiple of **3-200** coils, that wrap around the limb or other anatomical part to be treated, forming a magnetic field very similar to that of an ideal solenoid, with all its related advantages, especially a highly uniform space field; furthermore, the large number of wires **52**B and coils allows to reduce the wire diameter, increasing flexibility, wearability, and ease of incorporation into a garment.**[146]** The wires **52**B of the electromagnetic distribution element **5000** are preferably Litz wires.

Each Litz wire preferably comprises a plurality of electrically conductive elementary wires, for example, made of a suitable copper or aluminium alloy, arranged side by side, for example helically wound around each other, and preferably electrically insulated from each other.

Advantageously, each elementary wire has an average diameter equal to or lower than **1.0** millimetre, and more preferably equal to or less than **0.70** millimetres, **0.50** millimetres, **0.30** millimetres, **0.20** millimetres, or **0.10** millimetres, thereby making it more flexible and thus more comfortable and convenient when worn.

Each Litz wire can comprise a number of elementary wires between **3** and **40**, or between **5** and **30**, between **10** and **26,** or between **11** and **15,** for example equal to **13** elementary wires or **26** elementary wires.

The electric generator **3000** is preferably configured for powering the at least one electromagnetic distribution element **5000** so that it generates a variable electromagnetic field in one of the following ways: substantially sinusoidal, according to a single halfwave rectified sine wave, or according to a double halfwave rectified sine wave.

The therapeutical device **1000** preferably comprises an anatomical support **7000** adapted to be worn or otherwise applied to a human or animal body so that, upon activating the electrical generator **3000,** this and/or the electromagnetic distribution element **5000** creates said electromagnetic field within the area where such body to be treated **P** is positioned, and the electromagnetic distribution element **3000** is preferably fixed to the anatomical support **7000** (Figures **4-9A**)**.**

The anatomical support **7000** can comprise one or more of the following elements: a membrane, a plate, a shell, a bracelet, a shoe, an insole, an orthopedic foot support or insert, a sock (Figures **4****,** **5**), a knee-high sock, a sleeve, a glove, shorts, pants (Figure **6**), underwear, a tank top, a t-shirt or other type of short- or long-sleeved t-shirt, a jersey (Figures **7****,** **8****,** **9**), a sweater preferably with long sleeves (Figures **7****,** **8****,** **9**), a sweatshirt (Figures **7****,** **8****,** **9**), a scarf, a collar, a collar, a bib, a lumbar strap.

The electromagnetic distribution element **5000** is advantageously at least partially inserted into the anatomical support **7000.**

The electromagnetic distribution element **5000** can, for example, be sewn into the anatomical support **7000** or inserted into one or more appropriate internal pockets which are preferably reversibly openable from the outside, for example by buttons, zippers, Velcro fasteners, or other hook-and-loop systems or ties, when the anatomical support **7000** comprises a garment (Figures **6-9**)**.**

Advantageously, when comprising a garment, the anatomical support **7000** is composed of a plurality of segments or other garment pieces **SG6.1, SG6.2, SG6.3; SG7.1, SG7.2, SG7.3, SG7.4, SG7.5; SG8.1, SG8.2, SG8.3; SG9.1, SG9.2, SG9.3** which can be reversibly fixed to each other by reversible fixing systems **7001** such as for example buttons, zippers (Figures **6-9**), Velcro fasteners, other hook-and-loop systems, or ties (Figures **6**A, **7**A, **8**A, **9**A).

Such segments can, for example, be pant leg sections, such as for example segments **SG6.2, SG6.3,** sleeve sections, such as for example segments **SG7.3, SG8.2, SG8.3, SG9.3;** sleeve caps adapted to accommodate a shoulder and the most proximal area of an arm, such as for example segment **SG9.2;** or even can form a shortsleeved t-shirt or jersey such as segment **SG8.1,** a tank top such as for example segment **SG9.1,** a pair of shorts such as for example segment **SG6.1,** or a sleeve adapted to accommodate a torso section of the user, such as for example segments **SG7.1, SG7.2, SG7.4, SG7.5.**

The anatomical support **7000** can comprise one or more segments in which an electromagnetic distribution element **5000** is housed, such as for example segments **SG6.2, SG7.2, SG8.2, SG9.2.**

Electromagnetic distribution elements **5000** incorporated into a garment greatly facilitate therapeutical or analgesic treatments that can last several hours, making them more effective: the user can indeed move and carry out activities at home or outside while wearing the anatomical support **7000.**

As, for example, in the embodiment of Figures **4** and **5****,** the therapeutical device **1000** is advantageously provided with a logic unit **11** programmed or otherwise adapted to control the electrical current that, flowing through wires **52**b or other electromagnetic distribution element **5,** generates the magnetic field that promotes the healing of the patient or otherwise the well-being of the treated subject wearing the device **1.**

The logic unit **11** advantageously contains one or more associations between various diseases or other disorders and the related electromagnetic emission patterns intended to treat or otherwise alleviate them.

Advantageously, logic unit **11** can have stored or otherwise archived one or more of the associations in the following Tables, which are useful for treating diseases or other disorders in acute, subacute, and/or chronic phases.

In the following Tables, "minimum treatment time" refers to the total treatment time, including any pause periods during which the currents and voltages powering the electromagnetic distribution element **5** are substantially zero.

Table **1** describes combinations of times, frequencies, and currents for treating, for example, the neck and head by an electromagnetic distribution element **5000,** which can be incorporated, for example, into a ruff, scarf, or collar.

**- Table 1 -**

| | | | | | | | |
|---|---|---|---|---|---|---|---|
| Inflammation diseases - Head and neck | Time [minute s] | **ACUTE** phase | | **SUBACUTE** phase | | **CHRONIC/MAINT ENANCE** phase | |

| | | Hertz | Gauss | Hertz | Gauss | Hertz | Gauss |
|---|---|---|---|---|---|---|---|
| Headaches and migraines | **180** | **5** | **20** | **10** | **40** | **20** | **60** |
| Sinusitis and otitis | **180** | **10** | **20** | **20** | **40** | **40** | **60** |
| Dental neuralgia | **240** | **5** | **20** | **10** | **40** | **20** | **60** |
| Trigeminal neuralgia | **240** | **5** | **20** | **10** | **40** | **20** | **60** |
| Neuralgia of other facial nerves | **240** | **5** | **20** | **10** | **40** | **20** | **60** |
| Upper respiratory tract inflammation | **240** | **10** | **20** | **20** | **40** | **40** | **60** |
| Dysphagia and vocal cord inflammation | **240** | **10** | **20** | **20** | **40** | **40** | **60** |
| Sleep disorders | **360** | **10** | **30** | **20** | **50** | **40** | **60** |
| Myotensive cervical pain | **180** | **10** | **20** | **30** | **40** | **50** | **60** |
| Myotensive cervicalbrachial pain | **180** | **10** | **20** | **30** | **40** | **50** | **60** |
| Post-fracture vertebral conditions | **240** | **20** | **30** | **40** | **50** | **50** | **60** |
| Sprains (whiplash) | **180** | **10** | **20** | **30** | **40** | **50** | **60** |
| Osteoarthritis | **240** | **20** | **30** | **40** | **50** | **50** | **60** |
| Discopathies | **240** | **20** | **30** | **40** | **50** | **50** | **60** |

The following Tables **2**, **2**A, and **2**B describe combinations of times, frequencies, and currents for treating, for example, the torso by an electromagnetic distribution element **5000,** which can be incorporated, for example, into a t-shirt, sweater, or shirt.

**- Table 2 -**

| **Diseases of upper torso or upper respiratory tract** | Time min | **ACUTE** phase | | **SUBACUTE** phase | | **CHRONIC/MAIN** TENANCE phase | |
|---|---|---|---|---|---|---|---|
| | | Hertz | Gauss | Hertz | Gauss | Hertz | Gauss |
| Tietze's syndrome | **180** | **20** | **40** | **40** | **50** | **50** | **60** |
| Sternoclavicular joint inflammation | **180** | **20** | **40** | **40** | **50** | **50** | **60** |
| Bronchopathies and bronchial asthma | **240** | **10** | **30** | **20** | **40** | **50** | **60** |
| Respiratory insufficiency | **240** | **10** | **30** | **20** | **40** | **50** | **60** |
| Sleep apnoea | **360** | **10** | **30** | **20** | **40** | **50** | **60** |
| Fractures of clavicle, sternum, rib, scapula | **240** | **30** | **40** | **40** | **60** | **60** | **80** |
| Intercostal neuralgia | **240** | **10** | **30** | **20** | **40** | **50** | **60** |
| Herpes zoster (Shingles) | **240** | **10** | **30** | **20** | **40** | **50** | **60** |
| Rib, cartilage and sternocostal contusions | **240** | **30** | **40** | **40** | **50** | **60** | **80** |
| Gastroesophageal reflux | **180** | **20** | **40** | **40** | **50** | **50** | **60** |
| Hiatal hernia | **180** | **20** | **40** | **40** | **50** | **50** | **60** |
| Gastritis and gastric pain | **180** | **20** | **40** | **40** | **50** | **50** | **60** |
| Gastroduodenitis | **180** | **20** | **40** | **40** | **50** | **50** | **60** |
| Gastro-duodenal ulcer | **180** | **10** | **30** | **30** | **50** | **40** | **50** |

**- Table 2A -**

| **Inflammation diseases of the upper digestive tract** | Time min | **ACUTE** phase | | **SUBACUTE** phase | | **CHRONIC/MAIN** TENANCE phase | |
|---|---|---|---|---|---|---|---|
| | | Hertz | Gauss | Hertz | Gauss | Hertz | Gauss |
| Gastroesophageal reflux | **180** | **20** | **40** | **40** | **50** | **50** | **60** |
| Hiatal hernia | **180** | **20** | **40** | **40** | **50** | **50** | **60** |
| Gastritis and gastric pain | **180** | **20** | **40** | **40** | **50** | **50** | **60** |
| Gastroduodenitis | **180** | **20** | **40** | **40** | **50** | **50** | **60** |
| Gastro-duodenal ulcer | **180** | **10** | **30** | **30** | **50** | **40** | **50** |

**- Table 2B -**

| Inflammati on diseases of the lower digestive tract | Time [min] | **ACUTE** phase | | **SUBACUTE** phase | | **CHRONIC/MAIN** TENANCE phase | |
|---|---|---|---|---|---|---|---|
| | | Hertz | Gauss | Hertz | Gauss | Hertz | Gauss |
| Colitis | 240 | 20 | 40 | 40 | 50 | 50 | 60 |
| Crohn's Disease | **240** | **10** | **30** | **30** | **50** | **40** | **50** |
| Colon diseases | **240** | **10** | **30** | **30** | **50** | **40** | **50** |
| Diarrhoea | **240** | **20** | **40** | **40** | **50** | **50** | **60** |
| Constipation | **240** | **20** | **40** | **40** | **50** | **50** | **60** |
| Sluggish bowel/lazy bowel | **240** | **20** | **40** | **40** | **50** | **50** | **60** |
| Dysbiosis, swelling, flatulence | **240** | **20** | **40** | **40** | **50** | **50** | **60** |

The following Table **3** describes combinations of times, frequencies, and currents for treating, for example, the torso by an electromagnetic distribution element **5000,** which can be incorporated, for example, into a t-shirt or sweater that covers the lower torso and abdomen.

**- Table 3 -**

| Inflammation diseases of the male and female urogenital tract | Time min | **ACUTE** phase | | **SUBACUTE** phase | | **CHRONIC** / MAIN TENANCE phase | |
|---|---|---|---|---|---|---|---|
| | | Hertz | Gauss | Hertz | Gauss | Hertz | Gauss |
| Cystitis | 180 | 10 | 20 | 20 | 40 | 40 | 60 |
| Dysmenorrhea - irregular menstrual cycle | **180** | **10** | **20** | **20** | **40** | **40** | **60** |
| Prostatic inflammation | **180** | **10** | **20** | **20** | **40** | **40** | **60** |
| Uterine and bladder prolapse | **240** | **10** | **20** | **20** | **40** | **40** | **60** |
| Haemorrhoids and anal fissures | **240** | **10** | **20** | **20** | **40** | **40** | **60** |

The following Table **4** describes combinations of times, frequencies, and currents for treating, for example, the torso by an electromagnetic distribution element **5000,** which can be incorporated, for example, into a t-shirt or sweater that covers the central part of the torso.

**- Table 4 -**

| **Painful diseases of the cervical, thoracic, and lumbosacral spine of traumatic and non-traumatic origin, and postural alterations with reduced functionality** | Time min | **ACUTE** phase | | **SUBACUTE** phase | | | **CHRONIC/MAIN** TENANCE phase |
|---|---|---|---|---|---|---|---|
| | | Hertz | Gauss | Hertz | Gauss | Hertz | Gauss |
| Vertebral fractures | **240** | **20** | **30** | **40** | **50** | **50** | **60** |
| Sprains (whiplash) | **180** | **10** | **20** | **30** | **40** | **50** | **60** |
| Osteoarthritis | **240** | **20** | **30** | **40** | **50** | **50** | **60** |
| Myotensive cervicalbrachialgia | **180** | **10** | **20** | **30** | **40** | **50** | **60** |
| Herniated disc | **180** | **10** | **20** | **30** | **40** | **50** | **60** |
| Discopathies | **240** | **20** | **30** | **40** | **50** | **50** | **60** |
| Osteoporosis | **240** | **20** | **30** | **40** | **60** | **60** | **80** |
| Osteopenia | **240** | **20** | **30** | **40** | **60** | **60** | **80** |
| Outcomes of fractures in general | **240** | **20** | **30** | **40** | **60** | **60** | **80** |
| Low back pain - cruralgia - sciatica | **180** | **10** | **20** | **20** | **50** | **50** | **60** |
| Vertebral instability | **240** | **20** | **30** | **40** | **60** | **50** | **60** |
| Spondylolysis | **240** | **20** | **30** | **40** | **60** | **50** | **60** |
| Overload and myotensive disorders | **180** | **10** | **20** | **30** | **40** | **50** | **60** |

The following Table **5** describes combinations of times, frequencies, and currents for treating, for example, the torso by an electromagnetic distribution element **5000,** which can be incorporated, for example, into a t-shirt or sweater that covers the lower part of the torso and the pelvis.

**- Table 5 -**

| Diseases | Time min | **ACUTE** phase Hertz Gauss | | **SUBACUTE** phase Hertz Gauss | | **CHRONIC/MAINTENANCE** phase Hertz Gauss | |
|---|---|---|---|---|---|---|---|
| Bone fractures | **240** | **20** | **30** | **40** | **50** | **50** | **60** |
| Trauma | **240** | **20** | **30** | **40** | **50** | **50** | **60** |
| Coccydynia | **240** | **10** | **20** | **20** | **40** | **40** | **50** |
| Pubalgia | **240** | **10** | **20** | **20** | **40** | **40** | **50** |
| Sacroiliitis | **240** | **10** | **20** | **20** | **40** | **40** | **50** |
| Piriformis syndrome | **180** | **20** | **30** | **40** | **50** | **50** | **60** |
| Birth-related trauma | **240** | **20** | **30** | **40** | **50** | **50** | **60** |
| Hip osteoarthritis | **240** | **20** | **30** | **30** | **50** | **50** | **70** |
| Coxalgia | **240** | **10** | **20** | **20** | **40** | **30** | **60** |
| Femoralacetabular impingement | **240** | **10** | **20** | **20** | **40** | **30** | **50** |
| Femoral fractures | **240** | **10** | **20** | **20** | **40** | **30** | **60** |
| Coxo-femoral dislocations | **240** | **10** | **20** | **20** | **40** | **30** | **50** |

The following Table **6** describes combinations of times, frequencies, and currents for treating, for example, a leg by an electromagnetic distribution element **5000**, which can be incorporated, for example, into a long or short pants leg or for example a long sock.

**- Table 6 -**

| Diseases | Time min | **ACUTE** phase | | **SUBACUTE** phase | | **CHRONIC/MAIN** TENANCE phase | |
|---|---|---|---|---|---|---|---|
| | | Hertz | Gauss | Hertz | Gauss | Hertz | Gauss |
| Muscular contractures | **240** | **20** | **40** | **40** | **50** | **30** | **60** |
| Muscle strains and tears | **180** | **20** | **40** | **40** | **50** | **50** | **60** |
| Sprains involving ligament injuries | **240** | **20** | **30** | **40** | **50** | **50** | **60** |
| ACL and other ligament injuries | **240** | **20** | **30** | **40** | **50** | **50** | **60** |
| Meniscal lesions | **240** | **20** | **30** | **40** | **50** | **50** | **60** |
| Cartilage diseases | **300** | **20** | **30** | **40** | **50** | **50** | **60** |
| Patellofemoral chondropathy | **300** | **20** | **30** | **40** | **50** | **50** | **60** |
| Osteomyelitis | **300** | **20** | **30** | **40** | **50** | **50** | **80** |
| Patellar tendon inflammation and pes anserinus | **180** | **30** | **30** | **40** | **50** | **50** | **60** |
| Periostitis | **300** | **20** | **30** | **40** | **50** | **50** | **80** |
| Tibia-fibula fracture | **240** | **20** | **30** | **40** | **50** | **50** | **80** |

The following Table **7** describes combinations of times, frequencies, and intensities for treating, for example, a foot by an electromagnetic distribution element **5000**, which can be incorporated, for example, into a short or long sock, a shoe, or a slipper.

**- Table 7 -**

| Diseases | Time min | **ACUTE** phase | | **SUBACUTE** phase | | **CHRONIC/MAIN** TENANCE phase | |
|---|---|---|---|---|---|---|---|
| | | Hertz | Gauss | Hertz | Gauss | Hertz | Gauss |
| Heel pain | **240** | **10** | **20** | **20** | **30** | **40** | **60** |
| Plantar fasciitis | **180** | **15** | **25** | **30** | **40** | **40** | **70** |
| Calcaneal spur | **240** | **20** | **30** | **40** | **50** | **50** | **80** |
| Hallux valgus | **240** | **15** | **25** | **30** | **50** | **40** | **60** |
| Metatarsalgia | **240** | **20** | **30** | **40** | **50** | **60** | **80** |
| Morton's neuroma | **180** | **10** | **20** | **20** | **40** | **40** | **60** |
| Tarsal tunnel syndrome | **240** | **15** | **25** | **30** | **50** | **50** | **80** |
| Post-traumatic outcomes of tibial-tarsal sprains | **300** | **10** | **25** | **30** | **60** | **40** | **80** |
| Functional overload of the tarsometatars al region | **180** | **15** | **25** | **40** | **60** | **60** | **80** |
| Post-fracture outcomes of foot and ankle bones | **360** | **20** | **30** | **40** | **60** | **60** | **80** |

The following Table **8** describes combinations of times, frequencies, and currents for treating, for example, a shoulder, an arm, or a hand by an electromagnetic distribution element **5000**, which can be incorporated, for example, into a sleeve, a shirt, a sweater or a glove.

**- Table 8 -**

| **Diseases** | Time min | **ACUTE** phase Hertz Gauss | | **SUBACUTE** phase Hertz Gauss | | **CHRONIC**/MAIN TENANCE phase Hertz Gauss | |
|---|---|---|---|---|---|---|---|
| | | | | | | | |
| Periarthritis | **180** | **10** | **20** | **20** | **40** | **40** | **80** |
| Rotator cuff injuries | **240** | **10** | **20** | **20** | **40** | **40** | **60** |
| Glenohumeral joint dislocations | **240** | **10** | **20** | **20** | **40** | **40** | **60** |
| Fractures of the humeral head or neck | **240** | **10** | **20** | **30** | **40** | **40** | **60** |
| Subacromial impingement syndrome | **180** | **20** | **30** | **40** | **50** | **50** | **80** |
| Adhesive capsulitis (frozen shoulder) | **240** | **20** | **30** | **40** | **50** | **50** | **80** |
| Tendinitis | **180** | **20** | **30** | **40** | **40** | **50** | **80** |
| Epicondylitis | **180** | **20** | **30** | **40** | **50** | **50** | **60** |
| Epitrochleitis | **180** | **20** | **30** | **40** | **50** | **50** | **60** |
| Cubital tunnel syndrome | **180** | **10** | **25** | **30** | **40** | **50** | **60** |
| Post-traumatic outcomes of complex fractures | **240** | **10** | **30** | **30** | **50** | **50** | **80** |
| | | | | | | | |
| Post-fracture outcomes of radius and ulna | **240** | **10** | **30** | **30** | **50** | **50** | **80** |
| Trigger finger | **180** | **20** | **30** | **30** | **50** | **50** | **60** |
| Carpal tunnel syndrome | **180** | **10** | **30** | **25** | **50** | **40** | **60** |
| Rhizarthrosis | **240** | **10** | **25** | **30** | **50** | **40** | **60** |
| Metacarpophal angeal osteoarthritis | **240** | **15** | **30** | **40** | **60** | **60** | **80** |
| Interphalangeal osteoarthritis | **240** | **15** | **30** | **40** | **60** | **60** | **80** |
| Post-fracture outcomes of hand bones | **360** | **20** | **30** | **50** | **60** | **60** | **80** |
| Post-fracture outcomes of wrist and radius and ulna epiphysis | **360** | **20** | **30** | **50** | **60** | **60** | **80** |

The therapeutical device **1** can clearly emit magnetic fields having effective and/or peak intensities, and correspondingly effective and/or peak magnetic flux densities, varying up to ±**3.0**%, or up to **±5.0%,** ±**10.0**%, ±**15.0**%, **±20.0%,** ±**25.0**%, or ±**30.0**% of the values indicated in Tables; in other words, the therapeutical device **1, 1'** can emit magnetic fields having effective or peak intensities, and correspondingly effective or peak magnetic flux densities, that deviate from the values indicated in Tables by up to ±**3.0**%, ±**5.0**%, ±**10.0**%, ±**15.0**%, ±**20.0**%, ±**25.0**%, or ±**30.0**%; for example, if one of the Tables indicates **80** Gauss, the therapeutical device **1, 1'** can emit magnetic fields having an intensity between **56** and **104** Gauss.

In general, a therapeutical device **1** according to specific embodiments of the invention can be configured for operating with values of frequency, magnetic flux density, and minimum treatment time within ±**0.03** times, or ±**0.05** times, ±**0.10** times, ±**0.15** times, ±**0.20** times, ±**0.25** times, or ±**0.30** times the respective value indicated in the previous tables, or it can be adapted to operate with values of frequency, magnetic flux density, and minimum treatment time that deviate from the values indicated in the Tables by up to ±**0.03,** ±**0.05, ±0.10, ±0.15, ±0.20, ±0.25, or ±0.30** times the respective value indicated in the Tables themselves; for example, if one of the Tables indicates **80** Gauss, the therapeutical device **1** can emit magnetic fields having an intensity between **56** and **104** Gauss.

The therapeutical device **1000** allows to treat and effectively solve numerous diseases or disorders in a non-invasive manner, by deep action on tissues when necessary.

For this purpose, the previously described combinations of time, frequency, and current intensity have proven to be particularly effective.

The inflammation detection system **1** can be advantageously used to promptly identify an inflammation condition that has not yet been perceived by a subject and to detect whether and how to treat it, for example, by the therapeutical device **1000,** or to modify, more precisely select, and refine the electromagnetic radiation therapeutical treatment delivered by the therapeutical device **1000.**

Possibly, the anatomical support **7000** can incorporate or contain, or be adapted to incorporate or contain, the detection unit **2**, **2**'.

An example of possible operation and use of the inflammation detection system **1** is now described.

A user who wants to know whether he/she has a latent or early-stage inflammation, for example in a shoulder, can apply, for example, the calibration unit **4** to his/her wrist, whose calibration anatomical interface **5,** as mentioned, can have the form of a bracelet.

The sensors of the calibration unit **4** acquire body parameters of the user or patient at the wrist or other anatomical part believed to be non-inflamed.

The user can then apply the detection unit **2, 2'** to the shoulder or other anatomical part suspected of being inflamed, for example by pressing the foldable patch that forms the measurement anatomical interface **3, 3'** against the skin of the shoulder.

The detection system **1** can then calibrate the sensor detections of the detection unit **2, 2'** based on the detections of the calibration unit **4:** in this way, when determining a possible inflammation of the user's shoulder, for example, based on the temperature of various points on the shoulder, the system **1** can consider, for example, the fact that the user, at that time, even in the absence of local inflammation, has an overall higher or lower body temperature compared to other subjects or moments, for example, in the absence of local inflammation, the wrist temperature might be around **36.8°C** or respectively **35.9°C,** thereby allowing a much more precise identification of inflammation or measurement of the degree of inflammation in the shoulder by the detection unit **2, 2'.**

Once the process of calibrating the detection unit **2, 2'** is completed, such unit can detect, by its sensors, the body parameters of the shoulder or other suspected anatomical part of the user and then determines the degree of inflammation in the shoulder or other anatomical part, with much greater accuracy than without calibration.

Based on the detections of the unit **2,** the patient or otherwise final user can assess whether to use the therapeutical device **1000** or resort to other treatments.

The detection system **1** therefore allows to measure the degree of inflammation in body areas of a user, with multiple resulting advantages: the ability to archive, share, and compare data from multiple patients to measure therapies or conduct clinical studies, as well as to refine the inflammation diagnoses themselves and/or initiate therapy even in a preventive mode, for example by artificial intelligence tools.

In any case, even without relying on heavy artificial intelligence or numerical processing, the detection system **1** allows to detect latent, incipient, or otherwise barely or not at all perceived inflammatory states in the patient, allowing effective preventive treatments.

The exemplary embodiments previously described are susceptible to various modifications and variations without departing from the scope of protection of the present invention.

For example, the therapeutical device **1000** can be adapted for use not only on human patients but also on animals.

Any reference in this description to "an embodiment," "an exemplary embodiment," means that a particular feature or structure described in relation to such embodiment is comprised in at least one embodiment of the invention, and in particular in a specific variant of the invention as defined in a main claim.

The fact that such expressions appear in various steps of the description does not necessarily imply that they refer only to the same embodiment.

Furthermore, when a feature, element, or structure is described in relation to a particular embodiment, it is understood that it is within the competence of the skilled person to apply such feature, element, or structure to other embodiments.

Numerical references that differ only by different apexes (e.g., **21', 21", 21**^{III}) , unless otherwise specified, indicate different variants of an element with the same name.

Moreover, all details are replaceable by technically equivalent elements.

For example, the materials used, as well as the dimensions, can be any depending on technical requirements.

It should be understood that an expression such as "A comprises B, C, D" or "A is formed by B, C, D" also comprises and describes the particular case in which "A consists of B, C, D".

The expression "A comprises an element B", unless otherwise specified, is to be understood as "A comprises one or more elements *B"*.

References to a "first, second, third, ... n-th entity" are intended solely to distinguish one from the other, but the indication of the n-th entity does not necessarily imply the existence of the first, second, ... (n-**1**)-th entity.

The examples and lists of possible variants of the present application are to be understood as non-exhaustive lists.

## Claims

1. System **(1)** for detecting inflammation of an anatomical part of a human or animal body, comprising a plurality of sensors of at least two types, wherein said types are selected from the following list: a body temperature sensor **(300),** a muscular contracture and/or electromyographic sensor **(302),** a tumefaction and/or swelling and/or anomalous liquid infiltration sensor, a heart rate sensor **(500),** a blood haemoglobin oxygen saturation sensor **(502),** a skin perspiration sensor, a skin electrical conductance or electrodermal activity sensor, a sweating sensor, a sensor for detecting skin redness or, more generally, colour.

2. System according to claim **1**, comprising an anatomical interface (**3, 3', 5**) configured for fixing two or more of said sensors **(300, 302, 500, 502, 504)** to the body of a user, and two or more of said sensors **(300, 302, 500, 502, 504)** are fixed on the anatomical interface (**3, 3'**,**5**) at a distance from each other between **3** and **120** millimetres.

3. System according to claim **1** or **2**, comprising an anatomical interface (**3, 3', 5**) configured for fixing two or more of said sensors **(300, 302, 500, 502, 504)** to the body of a user, wherein the anatomical interface (3, **3**', **5**) forms one or more of the following elements: a patch, a bandage, a strap, a bracelet, a collar, a sleeve, a sock to be worn, for example, on a foot, a glove, a short- or long-sleeved t-shirt, a shirt, a gilet, a bib, underwear, short or long pants, a skirt, a hat, a cap, a membrane, a shell, a wristband, a knee brace, an elbow brace, an ankle brace, a finger ring or a finger cot.

4. System according to at least claim **2** and/or **3**, comprising at least one measurement anatomical interface **(3, 3')** and one calibration anatomical interface **(5),** wherein:
- the measurement anatomical interface **(3, 3')** is configured for being fixed to a first anatomical part of the body of the user, and the sensors of the measurement anatomical interface **(3, 3')** are configured for detecting one or more body parameters of the first anatomical part;
- the calibration anatomical interface **(5)** is configured for being fixed to a second anatomical part of the body of the user, and the sensors of the calibration anatomical interface **(5)** are configured for detecting one or more body parameters of the second anatomical part;
- the inflammation detection system **(1)** is configured for calibrating the detections of the sensors fixed to the measurement anatomical interface **(3, 3')** based on the detections of the sensors fixed to the calibration anatomical interface **(5).**

5. System according to at least claim **4**, wherein the sensors of the calibration anatomical interface **(5)** are adapted to detect, on the second anatomical part of the user's body, one or more body parameters substantially of the same type as the body parameters detected by the sensors of the measurement anatomical interface **(3, 3').**

6. System according to at least claim **4** or **5**, adapted to calibrate the detections of the sensors fixed to the measurement anatomical interface **(3, 3')** or to the detection unit **(2, 2'),** by deriving an index of a substantial absence of inflammation from the detections of one or more body parameters acquired by one or more sensors fixed to the calibration anatomical interface **(5)** or acquired by the calibration unit **(4).**

7. System according to at least one of claims **4** to **6**, adapted to determine the degree of inflammation of a first anatomical part based on the difference between a) the detections of at least one body parameter acquired on the first anatomical part of the user's body by the detection unit **(2, 2')** or by one or more sensors of the measurement anatomical interface **(3, 3');** and b) the detections of the body parameter of the same type acquired on the second anatomical part of the user's body by the calibration unit **(4)** or by one or more sensors fixed to the calibration anatomical interface (**5**).

8. System according to at least claim **7**, adapted to calibrate the detections of the sensors fixed to the measurement anatomical interface **(3, 3')** based on the difference between:
**a)** the value **Var_Par(**i**)_Tar** (k) detected by at least one sensor of the calibration anatomical interface **5;** and
**b)** the value **Var_Par(**i**)_Ril** (k) of the at least one i-th body parameter of the same type detected at the same k-th instant by at least one of the sensors of the measurement anatomical interface **(3, 3').**

9. System according to at least claim **7**, adapted to calibrate the detections of a plurality of sensors of the detection unit **2, 2'** based on the difference between:
**a)** the detections **Var_Par(1)_Tar(**h), **Var_Par(2)_Tar** (h) ... **Var_Par(**i)**_Tar(**h) ... **Var_Par(**N)**_Tar(**h) of a number N of sensors and/or relating to body parameters **1, 2,** ... i... N-th, acquired at the h-th instant by the calibration unit **4,** or otherwise by sensors fixed to the calibration anatomical interface **5,** on a second anatomical part; and
**b)** the detections **Var_Par(1)_Ril(k)**, **Var_Par(2)_Ril(k) ... Var_Par(i)_Ril_(k) ... Var_Par(N)_Ril(k)** of a corresponding number N of sensors and/or relating to **1, 2,** ... i... N corresponding body parameters, detections acquired at the k-th instant by the detection unit **2, 2'** or otherwise by sensors fixed to the measurement anatomical interface **3, 3'** on a first anatomical part, wherein i, h, k and N are integers equal to or greater than **1,** k is equal to or greater than h, and a detection **Var_Par_(**i)**_Tar(**h) relating to an i-th body parameter of the same type relates to the corresponding detection **Var_Par(**i)**_Ril_(**k**)** regardless of the value of h and k.

10. System according to one or more of the preceding claims, programmed or otherwise configured for determining:
**S5.1)** an acute inflammation state based on the detection of one or more of the following body parameters: temperature, tumefaction, liquid circulation, swelling, sweating, redness, haemoglobin oxygen saturation, muscular contracture, sweating, heart rate; and/or
**S5.2)** a subacute inflammation state based on the detections of one or more of the following body parameters: temperature, skin electrical conductivity or electrodermal activity;
**S5.3)** a chronic inflammation state at least based on the detection of the skin electrical conductivity or electrodermal activity, for example, the muscular contracture.

11. System according to one or more of the preceding claims, wherein said plurality of sensors **(300, 302, 500, 502, 504)** is programmed or otherwise adapted to determine the level of inflammation of an anatomical part by determining one or more of the following body parameters of the anatomical part: temperature, redness, tumefaction, anomalous liquid infiltration, pain, pathological or otherwise anomalous muscular contracture, skin electrical conductivity or electrodermal activity, heart rate, blood oxygenation.

12. System according to at least claim **4,** wherein the measurement anatomical interface **(3, 3')** comprises at least one muscular contracture and/or electromyographic sensor **(302).**

13. Therapeutical system comprising:
- a system for detecting inflammation **(1)** having the features according to one or more of claims **1-12;** and
- a therapeutical device **(1000)** comprising in turn at least one electrical generator **(3, 3'**) configured for generating a variable electromagnetic field with a frequency between **2** and **120** Hertz and a maximum or peak, or effective, magnetic induction between **5** and **160** Gauss, based on the detections of the one or more body parameters provided by the system for detecting inflammation **(1).**

14. Process for detecting an inflammation state, comprising the following operations:
**S15.1)** providing a system having the features according to at least claim **4;**
**S15.2)** by the calibration anatomical interface **(5)** detecting the body parameters of a first anatomical part of a user, which is presumed to be non-inflamed;
**S15.3)** by the measurement anatomical interface **(3)** detecting the body parameters of a second anatomical part of a user;
**S15.4)** calibrating the detections of the sensors fixed to the measurement anatomical interface **(3, 3')** based on the detections of the sensors fixed to the calibration anatomical interface **(5).**

15. Process according to claim **15,** wherein in operation **S15.4),** the detections of the sensors fixed to the measurement anatomical interface **(3, 3')** are calibrated by deriving an index of a substantial absence of inflammation from the detections of one or more body parameters acquired by one or more sensors fixed to the calibration anatomical interface **(5).**
